# EUROPEAN PATENT APPLICATION

(11) **EP 3 685 856 A1**
(43) Date of publication of application: **29.07.2020**
(21) Application number: 20156338.4
(22) Date of filing: 13.04.2016
(51) Int. Cl.: A61K 39/395, C07K 16/24, A61K 39/00, C07K 16/46, A61P 11/00, A61P 11/06

(54) **TREATMENT OF ASTHMA WITH ANTI-IL-23A ANTIBODIES**

(30) Priority: 14.04.2015 US 201562147281 P; 18.09.2015 US 201562220385 P
(62) Divisional of application: 18173790.9
(71) Applicant: Boehringer Ingelheim International GmbH, 55216 Ingelheim am Rhein (DE)
(72) Inventor: Arndt-Schmitz, Kirsten, 55216 Ingelheim am Rhein (DE); Berton, Alix, 55216 Ingelheim am Rhein (DE); Nivens, Michael Chadham, 55216 Ingelheim am Rhein (DE); Quast, Karsten, 55216 Ingelheim am Rhein (DE); Schlepuetz, Marco, 55216 Ingelheim am Rhein (DE); Visvanathan, Sudha, Ridgefield, CT 06877-0368 (US)
(74) Representative: Hoffmann Eitle

(57) **Abstract**

This invention generally relates to methods for the treatment of respiratory diseases, such as asthma, utilizing anti-IL-23A antibodies.

SEQUENCE LISTING

<110> BOEHRINGER INGELHEIM INTERNATIONAL GMBH

<120> METHODS OF TREATING DISEASES

<130> 09-0640-WO-1

<140>
<141>

<150> 62/220,385
<151> 2015-09-18

<150> 62/147,281
<151> 2015-04-14

<160> 22

<170> PatentIn version 3.5

<210> 1
<211> 11
<212> PRT
<213> Mus sp.

<400> 1
Lys Ala Ser Arg Asp Val Ala Ile Ala Val Ala
1               5                   10

<210> 2
<211> 7
<212> PRT
<213> Mus sp.

<400> 2
Trp Ala Ser Thr Arg His Thr
1               5

<210> 3
<211> 9
<212> PRT
<213> Mus sp.

<400> 3
His Gln Tyr Ser Ser Tyr Pro Phe Thr
1               5

<210> 4
<211> 10
<212> PRT
<213> Mus sp.

<400> 4
Gly Asn Thr Phe Thr Asp Gln Thr Ile His
1               5                   10

<210> 5
<211> 17

<212> PRT
<213> Mus sp.

<400> 5
Tyr Ile Tyr Pro Arg Asp Asp Ser Pro Lys Tyr Asn Glu Asn Phe Lys
1               5                   10                  15

Gly

<210> 6
<211> 11
<212> PRT
<213> Mus sp.

<400> 6
Pro Asp Arg Ser Gly Tyr Ala Trp Phe Ile Tyr
1               5                   10

<210> 7
<211> 10
<212> PRT
<213> Artificial Sequence

<220>
<221> source
<223> /note="Description of Artificial Sequence: Humanized
      sequence"

<400> 7
Gly Tyr Thr Phe Thr Asp Gln Thr Ile His
1               5                   10

<210> 8
<211> 10
<212> PRT
<213> Artificial Sequence

<220>
<221> source
<223> /note="Description of Artificial Sequence: Humanized
      sequence"

<400> 8
Gly Phe Thr Phe Thr Asp Gln Thr Ile His
1               5                   10

<210> 9
<211> 10
<212> PRT
<213> Artificial Sequence

<220>
<221> source
<223> /note="Description of Artificial Sequence: Humanized
      sequence"

<400> 9

Gly Gly Thr Phe Thr Asp Gln Thr Ile His
1               5                   10

<210> 10
<211> 107
<212> PRT
<213> Artificial Sequence

<220>
<221> source
<223> /note="Description of Artificial Sequence: Humanized
      sequence"

<400> 10
Asp Ile Gln Met Thr Gln Ser Pro Ser Ser Leu Ser Ala Ser Val Gly
1               5                   10                  15

Asp Arg Val Thr Ile Thr Cys Lys Ala Ser Arg Asp Val Ala Ile Ala
            20                  25                  30

Val Ala Trp Tyr Gln Gln Lys Pro Gly Lys Val Pro Lys Leu Leu Leu
        35                  40                  45

Phe Trp Ala Ser Thr Arg His Thr Gly Val Pro Asp Arg Phe Ser Gly
    50                  55                  60

Ser Gly Ser Gly Thr Asp Phe Thr Leu Thr Ile Ser Ser Leu Gln Pro
65                  70                  75                  80

Glu Asp Leu Ala Asp Tyr Tyr Cys His Gln Tyr Ser Ser Tyr Pro Phe
                85                  90                  95

Thr Phe Gly Gln Gly Thr Lys Leu Glu Ile Lys
            100                 105

<210> 11
<211> 107
<212> PRT
<213> Artificial Sequence

<220>
<221> source
<223> /note="Description of Artificial Sequence: Humanized
      sequence"

<400> 11
Asp Ile Gln Met Thr Gln Ser Pro Ser Ser Leu Ser Ala Ser Val Gly
1               5                   10                  15

Asp Arg Val Thr Ile Thr Cys Lys Ala Ser Arg Asp Val Ala Ile Ala
            20                  25                  30

Val Ala Trp Tyr Gln Gln Lys Pro Gly Lys Val Pro Lys Leu Leu Ile

35                  40                  45

Tyr Trp Ala Ser Thr Arg His Thr Gly Val Pro Ser Arg Phe Ser Gly
    50                  55                  60

Ser Gly Ser Arg Thr Asp Phe Thr Leu Thr Ile Ser Ser Leu Gln Pro
65                  70                  75                  80

Glu Asp Val Ala Asp Tyr Phe Cys His Gln Tyr Ser Ser Tyr Pro Phe
                85                  90                  95

Thr Phe Gly Ser Gly Thr Lys Leu Glu Ile Lys
            100                 105

<210> 12
<211> 107
<212> PRT
<213> Artificial Sequence

<220>
<221> source
<223> /note="Description of Artificial Sequence: Humanized
      sequence"

<400> 12
Asp Ile Gln Met Thr Gln Ser Pro Ser Ser Leu Ser Ala Ser Val Gly
1               5                   10                  15

Asp Arg Val Thr Ile Thr Cys Lys Ala Ser Arg Asp Val Ala Ile Ala
            20                  25                  30

Val Ala Trp Tyr Gln Gln Lys Pro Gly Lys Val Pro Lys Leu Leu Leu
        35                  40                  45

Tyr Trp Ala Ser Thr Arg His Thr Gly Val Pro Ser Arg Phe Ser Gly
    50                  55                  60

Ser Gly Ser Arg Thr Asp Phe Thr Leu Thr Ile Ser Ser Leu Gln Pro
65                  70                  75                  80

Glu Asp Val Ala Thr Tyr Tyr Cys His Gln Tyr Ser Ser Tyr Pro Phe
                85                  90                  95

Thr Phe Gly Ser Gly Thr Lys Leu Glu Ile Lys
            100                 105

<210> 13
<211> 107
<212> PRT
<213> Artificial Sequence

<220>
<221> source
<223> /note="Description of Artificial Sequence: Humanized
      sequence"

<400> 13
Asp Ile Gln Met Thr Gln Ser Pro Ser Ser Leu Ser Ala Ser Val Gly
1               5                   10                  15

Asp Arg Val Thr Ile Thr Cys Lys Ala Ser Arg Asp Val Ala Ile Ala
            20                  25                  30

Val Ala Trp Tyr Gln Gln Lys Pro Gly Lys Val Pro Lys Leu Leu Leu
        35                  40                  45

Phe Trp Ala Ser Thr Arg His Thr Gly Val Pro Asp Arg Phe Ser Gly
    50                  55                  60

Ser Gly Ser Arg Thr Asp Phe Thr Leu Thr Ile Ser Ser Leu Gln Pro
65                  70                  75                  80

Glu Asp Leu Ala Asp Tyr Tyr Cys His Gln Tyr Ser Ser Tyr Pro Phe
                85                  90                  95

Thr Phe Gly Ser Gly Thr Lys Leu Glu Ile Lys
            100                 105


<210> 14
<211> 120
<212> PRT
<213> Artificial Sequence

<220>
<221> source
<223> /note="Description of Artificial Sequence: Humanized
      sequence"

<400> 14
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asp Gln
            20                  25                  30

Thr Ile His Trp Met Arg Gln Ala Pro Gly Gln Gly Leu Glu Trp Ile
        35                  40                  45

Gly Tyr Ile Tyr Pro Arg Asp Asp Ser Pro Lys Tyr Asn Glu Asn Phe
    50                  55                  60

Lys Gly Lys Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Pro Asp Arg Ser Gly Tyr Ala Trp Phe Ile Tyr Trp Gly Gln
            100                 105                 110

Gly Thr Leu Val Thr Val Ser Ser
        115                 120

<210> 15
<211> 120
<212> PRT
<213> Artificial Sequence

<220>
<221> source
<223> /note="Description of Artificial Sequence: Humanized
      sequence"

<400> 15
Gln Val Gln Leu Val Gln Ser Gly Ala Glu Val Lys Lys Pro Gly Ser
1               5                   10                  15

Ser Val Lys Val Ser Cys Lys Ala Ser Gly Phe Thr Phe Thr Asp Gln
            20                  25                  30

Thr Ile His Trp Val Arg Gln Ala Pro Gly Gln Gly Leu Glu Trp Met
        35                  40                  45

Gly Tyr Ile Tyr Pro Arg Asp Asp Ser Pro Lys Tyr Asn Glu Asn Phe
    50                  55                  60

Lys Gly Lys Val Thr Leu Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Pro Asp Arg Ser Gly Tyr Ala Trp Phe Ile Tyr Trp Gly Gln
            100                 105                 110

Gly Thr Leu Val Thr Val Ser Ser
        115                 120

<210> 16
<211> 120
<212> PRT
<213> Artificial Sequence

<220>

<221> source
<223> /note="Description of Artificial Sequence: Humanized
      sequence"

<400> 16
Gln Val Gln Leu Val Gln Ser Gly Ala Glu Val Lys Lys Pro Gly Ser
1               5                   10                  15

Ser Val Lys Thr Ser Cys Lys Ala Ser Gly Gly Thr Phe Thr Asp Gln
            20                  25                  30

Thr Ile His Trp Val Arg Gln Arg Pro Gly Gln Gly Leu Glu Trp Met
        35                  40                  45

Gly Tyr Ile Tyr Pro Arg Asp Asp Ser Pro Lys Tyr Asn Glu Asn Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Pro Asp Arg Ser Gly Tyr Ala Trp Phe Ile Tyr Trp Gly Gln
            100                 105                 110

Gly Thr Leu Val Thr Val Ser Ser
        115                 120


<210> 17
<211> 120
<212> PRT
<213> Artificial Sequence

<220>
<221> source
<223> /note="Description of Artificial Sequence: Humanized
      sequence"

<400> 17
Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Thr Asp Gln
            20                  25                  30

Thr Ile His Trp Val Arg Gln Ala Pro Gly Gln Gly Leu Glu Trp Met
        35                  40                  45

Gly Tyr Ile Tyr Pro Arg Asp Asp Ser Pro Lys Tyr Asn Glu Asn Phe
    50                  55                  60

Lys Gly Arg Val Thr Leu Thr Ala Asp Lys Ser Thr Ser Thr Ala Tyr
65                  70                  75                  80

Met Glu Leu Ser Ser Leu Arg Ser Glu Asp Thr Ala Val Tyr Phe Cys
                85                  90                  95

Ala Arg Pro Asp Arg Ser Gly Tyr Ala Trp Phe Ile Tyr Trp Gly Gln
            100                 105                 110

Gly Thr Leu Val Thr Val Ser Ser
        115                 120

<210> 18
<211> 214
<212> PRT
<213> Artificial Sequence

<220>
<221> source
<223> /note="Description of Artificial Sequence: Humanized
      sequence"

<400> 18
Asp Ile Gln Met Thr Gln Ser Pro Ser Ser Leu Ser Ala Ser Val Gly
1               5                   10                  15

Asp Arg Val Thr Ile Thr Cys Lys Ala Ser Arg Asp Val Ala Ile Ala
            20                  25                  30

Val Ala Trp Tyr Gln Gln Lys Pro Gly Lys Val Pro Lys Leu Leu Ile
        35                  40                  45

Tyr Trp Ala Ser Thr Arg His Thr Gly Val Pro Ser Arg Phe Ser Gly
    50                  55                  60

Ser Gly Ser Arg Thr Asp Phe Thr Leu Thr Ile Ser Ser Leu Gln Pro
65                  70                  75                  80

Glu Asp Val Ala Asp Tyr Phe Cys His Gln Tyr Ser Ser Tyr Pro Phe
                85                  90                  95

Thr Phe Gly Ser Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 19
<211> 449
<212> PRT
<213> Artificial Sequence

<220>
<221> source
<223> /note="Description of Artificial Sequence: Humanized
      sequence"

<400> 19
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asp Gln
            20                  25                  30

Thr Ile His Trp Met Arg Gln Ala Pro Gly Gln Gly Leu Glu Trp Ile
        35                  40                  45

Gly Tyr Ile Tyr Pro Arg Asp Asp Ser Pro Lys Tyr Asn Glu Asn Phe
    50                  55                  60

Lys Gly Lys Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Pro Asp Arg Ser Gly Tyr Ala Trp Phe Ile Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Arg Val Glu Pro Lys Ser Cys Asp
    210                 215                 220

Lys Thr His Thr Cys Pro Pro Cys Pro Ala Pro Glu Ala Ala Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly

<210> 20
<211> 449
<212> PRT
<213> Artificial Sequence

<220>
<221> source
<223> /note="Description of Artificial Sequence: Humanized
      sequence"

<400> 20
Gln Val Gln Leu Val Gln Ser Gly Ala Glu Val Lys Lys Pro Gly Ser
1               5                   10                  15

Ser Val Lys Val Ser Cys Lys Ala Ser Gly Phe Thr Phe Thr Asp Gln
            20                  25                  30

Thr Ile His Trp Val Arg Gln Ala Pro Gly Gln Gly Leu Glu Trp Met
        35                  40                  45

Gly Tyr Ile Tyr Pro Arg Asp Asp Ser Pro Lys Tyr Asn Glu Asn Phe
    50                  55                  60

Lys Gly Lys Val Thr Leu Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Pro Asp Arg Ser Gly Tyr Ala Trp Phe Ile Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Arg Val Glu Pro Lys Ser Cys Asp
    210                 215                 220

Lys Thr His Thr Cys Pro Pro Cys Pro Ala Pro Glu Ala Ala Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp

370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly

<210> 21
<211> 214
<212> PRT
<213> Artificial Sequence

<220>
<221> source
<223> /note="Description of Artificial Sequence: Humanized
      sequence"

<400> 21
Asp Ile Gln Met Thr Gln Ser Pro Ser Ser Leu Ser Ala Ser Val Gly
1               5                   10                  15

Asp Arg Val Thr Ile Thr Cys Lys Ala Ser Arg Asp Val Ala Ile Ala
            20                  25                  30

Val Ala Trp Tyr Gln Gln Lys Pro Gly Lys Val Pro Lys Leu Leu Leu
        35                  40                  45

Phe Trp Ala Ser Thr Arg His Thr Gly Val Pro Asp Arg Phe Ser Gly
    50                  55                  60

Ser Gly Ser Gly Thr Asp Phe Thr Leu Thr Ile Ser Ser Leu Gln Pro
65                  70                  75                  80

Glu Asp Leu Ala Asp Tyr Tyr Cys His Gln Tyr Ser Ser Tyr Pro Phe
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly

115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 22
<211> 189
<212> PRT
<213> Homo sapiens

<400> 22
Met Leu Gly Ser Arg Ala Val Met Leu Leu Leu Leu Leu Pro Trp Thr
1               5                   10                  15

Ala Gln Gly Arg Ala Val Pro Gly Gly Ser Ser Pro Ala Trp Thr Gln
            20                  25                  30

Cys Gln Gln Leu Ser Gln Lys Leu Cys Thr Leu Ala Trp Ser Ala His
        35                  40                  45

Pro Leu Val Gly His Met Asp Leu Arg Glu Glu Gly Asp Glu Glu Thr
    50                  55                  60

Thr Asn Asp Val Pro His Ile Gln Cys Gly Asp Gly Cys Asp Pro Gln
65                  70                  75                  80

Gly Leu Arg Asp Asn Ser Gln Phe Cys Leu Gln Arg Ile His Gln Gly
                85                  90                  95

Leu Ile Phe Tyr Glu Lys Leu Leu Gly Ser Asp Ile Phe Thr Gly Glu
            100                 105                 110

Pro Ser Leu Leu Pro Asp Ser Pro Val Gly Gln Leu His Ala Ser Leu
        115                 120                 125

Leu Gly Leu Ser Gln Leu Leu Gln Pro Glu Gly His His Trp Glu Thr
    130                 135                 140

Gln Gln Ile Pro Ser Leu Ser Pro Ser Gln Pro Trp Gln Arg Leu Leu
145                 150                 155                 160

Leu Arg Phe Lys Ile Leu Arg Ser Leu Gln Ala Phe Val Ala Val Ala
                165                 170                 175

Ala Arg Val Phe Ala His Gly Ala Ala Thr Leu Ser Pro
            180                 185

## Description

### Technical Field of the Invention

This invention generally relates to method of treating respiratory diseases, for example asthma, utilizing anti-IL-23A antibodies.

### Background of the Invention

Asthma is a chronic inflammatory disease of the airways and characterized by various symptoms like increased mucus production, cough and dyspnea and wheezing as signs of airflow obstruction. The symptoms are caused by inflammation-induced hyperreactivity of the upper airways and may be lower airways. Around the globe around 300 millions people suffer from this disease. Among them an estimated 5-10% have severe asthma, driving the majority of healthcare utilization and costs associated with asthma.

While severe asthma is a significant health-economic problem, the availability of effective treatments in clinical practice remains limited. However, there is a growing realisation of the heterogeneity of asthma which is not a single condition, but composed of numerous potential phenotypes some of which may show association to more severe disease status. In this regard recent adoption of unbiased statistical techniques such as cluster analysis has demonstrated presence of severe asthma clusters in both adult and paediatric populations. These clinical phenotypes may show specific pathophysiological associations leading to classifications of endotypes. Accompanying this growing understanding of the diverse nature of asthma is the realisation that there are also a diverse range of treatment responses dependent on the underlying nature of an individual's disease.

The pathophysiology of asthma is heterogeneous and driven by genetic, epigenetic, environmental as well as unknown causes. Hallmarks are the involvement of various cell types like eosinophils, T-cell subsets, mast cells, basophils and neutrophils which are activated in response to allergic triggers, viral infection, and even bacterial colonization.

Due to the complexity of triggers and reactions of the immune system asthma can not be regarded as one phenotype only. Currently scientific as well as clinical efforts are directed towards identification of various subtypes of the disease which might reflect variable contributions from these cell types.

For example, one phenotype is represented by early onset disease already in children below the age of 12 associated with atopy which is a sensitization of the bodies reaction to allergens mediated by IgE. Here, eosinophilia in blood and bronchoalveolar lavage fluid (BALF) and/ or sputum as well as T-helper cell type 2 (Th2) and innate lymphoid type 2 cell (ILC2) responses are prevalent and can be identified by increased levels of the cytokines IL4, IL5 and IL13 and eotaxins. Other potential phenotypes are regarded as non-Th2 phenotypes and might have a later onset of disease i.e. in adulthood in early to mid forties (Wenzel S 2012 Clin Exp Allergy. 42:650-8). Smoking asthmatics are found in this group and characterized by an increased number of neutrophils in the airways.

Non-Th2 and/or non-eosinophil asthma might include a set of different cytokines as markers of the inflammatory process. Cytokines like IL17A and F have been shown to be released by a specific set of immune cells, i.e. T-helper type 17 cell (Th17 cells), innate lymphoid type 3 cell (ILC3), γδ-T, NK cells among others. A permissive environment for the development of Th17 cells is established by activation of T-cells together with the activation of various pattern recognition receptors, e.g. toll-like recepeptors (TLRs). In some cases high antigen concentrations are needed to trigger the initiation of Th17 cell development (Lezzi G et al., 2009 Proc Natl Acad Sci USA. 106:876-81). Under high antigen concentrations, cytokines (i.e. IL1β, IL6, TGFβ) are released which are able to drive the differentiation of T-cells towards the Th17 phenotype providing a sustained source of inflammation. In various tissues like the skin, the cytokine IL-23 seems to be important in expanding and maintaining the Th17 phenotype. Th17 cells specifically release cytokines like IL17A and F, IL22 which in turn induces the release of IL8 by epithelial cells which is an attractor of neutrophils to the lung.

Mild to moderate asthma is treated with anti-inflammatories like inhaled steroids, long and short acting beta-agonists, montelukast and theophylline. In the more severe populations oral steroids are added, but do not suffice to control all asthma phenotypes. There is therefore a medical need for the treatment of more severe forms of asthma, for example for phenotypes which display a high number of certain cell subpopulations and/or are steroid resistant.

### Summary of the Invention

The present invention addresses the above needs and provides methods for treating asthma, in particular severe persistent asthma, comprising administering an anti-IL-23A antibody to a patient.

In one aspect, a method of the present invention is for treating neutrophilic asthma. In one aspect, a method of the present invention is for treating eosinophilic asthma. In one aspect, a method of the present invention is for treating mixed neutrophilic and eosinophilic asthma. In one aspect, a method of the present invention is for treating paucigranulocytic asthma. In one aspect, asthma is categorized by induced sputum.

In one aspect, a patient in one of the above methods is not responsive to corticosteroids.

In one aspect, in one of the above methods, the treatment is assessed by the reduction of the annual rate of exacerbations in patients, for example patients with severe persistent asthma, for example with a hazard ratio (HR) of 0.75. In one aspect, in one of the above methods, the treatment is assessed by the relative improvement in proportion of patients having symptomatic improvements, for example an Asthma Control Questionnaire (ACQ) reduction of 0.5 units: 20 %. In one aspect, in one of the above methods, the treatment is assessed by improvement in trough Forced Expiratory Volume in 1 second (FEV₁), e.g. > 125ml, reduction of current controller therapies, improvement in health related QoL (e.g. Asthma Quality of Life Questionnaire (AQLQ)), or improvement in symptoms score / rescue medication use.

In one aspect, in one of the above methods, asthma is mediated by Th17 cells or cells responsive to IL-23. In one aspect, in one of the above methods, asthma is mediated by Th2 cells. In one aspect, in one of the above methods, asthma is mediated by Th2/Th17 dual-positive cells or cells responsive to Th2 responsive cytokines and IL-23.

In a further aspect, the present invention provides a method for the treatment or prophylaxis of asthma exacerbations comprising administering an anti-IL-23A antibody to a patient with asthma, in particular severe asthma, for example severe persistent asthma.

In one aspect of the present invention a patient is a patient who experiences frequent exacerbations and in a further embodiment, the present invention provides a method for improving exacerbations in patients who experience frequent exacerbations comprising administering an anti-IL-23A antibody to the patient with asthma, in particular severe asthma, for example severe persistent asthma.

In a further aspect, the present invention provides a method for the treatment or control of symptoms of severe asthma comprising administering an anti-IL-23A antibody to a patient with severe persistent asthma.

In a further aspect, the present invention provides a method for the treatment or prophylaxis of asthma exacerbations and for the treatment or control of symptoms of severe asthma comprising administering an anti-IL-23A antibody to a patient with severe persistent asthma.

In a further aspect, the present invention provides a method to reduce the rate of asthma exacerbations comprising administering an anti-IL-23A antibody to a patient with severe persistent asthma.

In one aspect, in one of the above methods, asthma is neutrophilic asthma. In one aspect, in one of the above methods, asthma is eosinophilic asthma. In one aspect, in one of the above methods, asthma is mixed neutrophilic and eosinophilic asthma. In one aspect, in one of the above methods, asthma is paucigranulocytic asthma. In one aspect, the type of asthma is categorized or phenotyped by induced sputum, by blood cell counts or by blood biomarkers which could be eosinophiles and/ or various cytokines or other proteins.

In one aspect, in one of the above methods, the patient is not adequately controlled on inhaled corticosteroids. In one aspect, in one of the above methods, the patient is not adequately controlled on inhaled corticosteroids and a second controller medication. In one aspect, in one of the above methods, the patient is 12 years of age and older. In one aspect, in one of the above methods, the patient is 12 years of age and older not adequately controlled on inhaled corticosteroids and a second controller medication.

In one aspect, in one the above methods, the treatment, prophylaxis, control or reduction is assessed by the reduction of annual rate of exacerbations in a patient, for example a patient with severe persistent asthma, for example with a hazard ratio (HR) of 0.75. In one aspect, in one the above methods, the treatment, prophylaxis, control or reduction is assessed by the relative improvement in proportion of patients having symptomatic improvements, for example a Asthma Control Questionnaire (ACQ) reduction of 0.5 units: 20 %. In one aspect, in one the above methods, the treatment, prophylaxis, control or reduction is assessed by improvement in trough FEV₁ (e.g. > 125ml), reduction of current controller therapies, improvement in health related QoL (e.g. Asthma Quality of Life Questionnaire (AQLQ)), or improvement in symptoms score / rescue medication use.

In one aspect, in one of the above methods, the anti-IL-23A antibody is Antibody A, Antibody B, Antibody C or Antibody D.

In one aspect, in one of the above methods, the anti-IL-23A antibody is administered to said patient by subcutaneous administration.

In one aspect, the present invention provides an anti-IL-23A antibody for use in a method described herein, for example in the treament of asthma, in particular severe persistent asthma, for example as described herein.

In one aspect, the present invention provides for the use of an anti-IL-23A antibody for the preparation of a medicament for the treatment of asthma, in particular severe persistent asthma, for example as described herein.

In one embodiment, in any one of the methods or uses above, the anti-IL-23A antibody is disclosed below.

In one embodiment, the anti-IL-23A antibody comprises a light chain variable region comprising the amino acid sequence of SEQ ID NO:1 *(CDR1-L);* the amino acid sequence of SEQ ID NO:2 *(CDR2-L);* and the amino acid sequence of SEQ ID NO:3 *(CDR3-L);* and a heavy chain variable region comprising the amino acid sequence of SEQ ID NO: 4, 7, 8 or 9 *(CDR1-H);* the amino acid sequence of SEQ ID NO:5 *(CDR2-*H); and the amino acid sequence of SEQ ID NO:6 *(CDR3-H).*

In one embodiment, the anti-IL-23A antibody comprises a light chain variable region comprising the amino acid sequence of SEQ ID NO:1 *(CDR1-L);* the amino acid sequence of SEQ ID NO:2 *(CDR2-L);* and the amino acid sequence of SEQ ID NO:3 *(CDR3-L);* and a heavy chain variable region comprising the amino acid sequence of SEQ ID NO:4 *(CDR1-H);* the amino acid sequence of SEQ ID NO:5 *(CDR2-H);* and the amino acid sequence of SEQ ID NO:6 *(CDR3-H).*

In one embodiment, the anti-IL-23A antibody comprises a light chain variable region comprising the amino acid sequence of SEQ ID NO:1 *(CDR1-L);* the amino acid sequence of SEQ ID NO:2 *(CDR2-L);* and the amino acid sequence of SEQ ID NO:3 *(CDR3-L);* and a heavy chain variable region comprising the amino acid sequence of SEQ ID NO:7 *(CDR1-H);* the amino acid sequence of SEQ ID NO:5 *(CDR2-H);* and the amino acid sequence of SEQ ID NO:6 *(CDR3-H).*

In one embodiment, the anti-IL-23A antibody comprises a light chain variable region comprising the amino acid sequence of SEQ ID NO:1 *(CDR1-L);* the amino acid sequence of SEQ ID NO:2 *(CDR2-L);* and the amino acid sequence of SEQ ID NO:3 *(CDR3-L);* and a heavy chain variable region comprising the amino acid sequence of SEQ ID NO:8 *(CDR1-H);* the amino acid sequence of SEQ ID NO:5 *(CDR2-H);* and the amino acid sequence of SEQ ID NO:6 *(CDR3-H).*

In one embodiment, the anti-IL-23A antibody comprises a light chain variable region comprising the amino acid sequence of SEQ ID NO:1 *(CDR1-L);* the amino acid sequence of SEQ ID NO:2 *(CDR2-L);* and the amino acid sequence of SEQ ID NO:3 *(CDR3-L);* and a heavy chain variable region comprising the amino acid sequence of SEQ ID NO:9 *(CDR1-H);* the amino acid sequence of SEQ ID NO:5 *(CDR2-H);* and the amino acid sequence of SEQ ID NO:6 *(CDR3-H).*

In one embodiment, the anti-IL-23A antibody comprises a light chain variable region comprising the amino acid sequence of any one of SEQ ID NO:10, 11, 12 or 13; and a heavy chain variable region comprising the amino acid sequence any one of SEQ ID NO:14, 15, 16 or 17.

In one embodiment, the anti-IL-23A antibody comprises a light chain variable region comprising the amino acid sequence of SEQ ID NO:11 and a heavy chain variable region comprising the amino acid sequence SEQ ID NO:14.

In one embodiment, the anti-IL-23A antibody comprises a light chain variable region comprising the amino acid sequence of SEQ ID NO:11 and a heavy chain variable region comprising the amino acid sequence SEQ ID NO:15.

In one embodiment, the anti-IL-23A antibody comprises a light chain variable region comprising the amino acid sequence of SEQ ID NO:10 and a heavy chain variable region comprising the amino acid sequence SEQ ID NO:14.

In one embodiment, the anti-IL-23A antibody comprises a light chain variable region comprising the amino acid sequence of SEQ ID NO:10 and a heavy chain variable region comprising the amino acid sequence SEQ ID NO:15.

In one embodiment, the anti-IL-23A antibody comprises the amino acid sequence SEQ ID NO:14 or 15 linked to a human IgG1, IgG2, IgG3, IgG4, IgM, IgA or IgE heavy chain constant region. In one embodiment, the anti-IL-23A antibody comprises the amino acid sequence of SEQ ID NO: 14 or 15 linked to a human IgG1 heavy chain constant region. In one embodiment, the anti-IL-23A antibody comprises the amino acid sequence of SEQ ID NO:10 or 11 linked to a human kappa or lambda light chain constant region.

In one embodiment, In one embodiment, the anti-IL-23A antibody comprises the amino acid sequence of SEQ ID NO:14 or 15 linked to a human IgG1 heavy chain constant region; and the amino acid sequence of SEQ ID NO: 10 or 11 linked to a human kappa light chain constant region.

In one embodiment, the anti-IL-23A antibody is a humanized monoclonal antibody comprising a light chain variable region comprising the amino acid sequence selected from the group consisting of any one of SEQ ID NO:10, 11, 12 and 13 and a heavy chain variable region comprising the amino acid sequence selected from the group consisting of any one of SEQ ID NO:14, 15, 16 and 17.

In one embodiment, the anti-IL-23A antibody is a humanized monoclonal antibody comprising a light chain variable region comprising the amino acid sequence of SEQ ID NO:11 and a heavy chain variable region comprising the amino acid sequence SEQ ID NO:14.

In one embodiment, the anti-IL-23A antibody is a humanized monoclonal antibody comprising a light chain variable region comprising the amino acid sequence of SEQ ID NO:11 and a heavy chain variable region comprising the amino acid sequence SEQ ID NO:15.

In one embodiment, the anti-IL-23A antibody is a humanized monoclonal antibody comprising a light chain variable region comprising the amino acid sequence of SEQ ID NO:10 and a heavy chain variable region comprising the amino acid sequence SEQ ID NO:14.

In one embodiment, the anti-IL-23A antibody is a humanized monoclonal antibody comprising a light chain variable region comprising the amino acid sequence of SEQ ID NO:10 and a heavy chain variable region comprising the amino acid sequence SEQ ID NO:15.

In one embodiment, the anti-IL-23A antibody comprises a light chain comprising the amino acid sequence of SEQ ID NO:18 or 21 and a heavy chain comprising the amino acid sequence of SEQ ID NO:19 or 20.

In one embodiment, the anti-IL-23A antibody comprises a light chain comprising the amino acid sequence of SEQ ID NO:18 and a heavy chain comprising the amino acid sequence of SEQ ID NO:19.

In one embodiment, the anti-IL-23A antibody comprises a light chain comprising the amino acid sequence of SEQ ID NO:18 and a heavy chain comprising the amino acid sequence of SEQ ID NO:20.

In one embodiment, the anti-IL-23A antibody comprises a light chain comprising the amino acid sequence of SEQ ID NO:21 and a heavy chain comprising the amino acid sequence of SEQ ID NO:19.

In one embodiment, the anti-IL-23A antibody comprises a light chain comprising the amino acid sequence of SEQ ID NO: 21 and a heavy chain comprising the amino acid sequence of SEQ ID NO: 20.

In one embodiment, the anti-IL-23A antibody is Antibody A, Antibody B, Antibody C or Antibody D.

In one embodiment, the anti-IL-23A antibody is as disclosed in WO2007/005955, WO2007/024846, WO2007/027714, WO2007/076524, WO2008/103432 or WO2012/061448.

### Brief Description of the Figures

Figure 1: Release of IL8 (CINC1) after treatment of rat precision-cut lung slices with IL-23 (median, interquartile range and 5-95% confidence interval).
Figure 2: Release of IL22 after treatment of rat precision-cut lung slices with IL-23 (median, interquartile range and 5-95% confidence interval).
Figure 3: IL22 release from rat precision-cut lung slices (PCLS) stimulated by IL23. Data are shown as scatter dot plot and mean ± SD is depicted. Replicates originate from n=5-6 rats. Means were compared by non-parametric Kruskal-Wallis test followed by Dunn's post-test.
Figure 4A and 4B: IL22 release from human PCLS stimulated by IL23 (A) or IL17 (B). Data are shown as scatter dot plot and mean ± SD is depicted. Replicates originate from n=3 human donors. Batches for each donor were performed in duplicates and supernatants were pooled before cytokine analysis. Means were compared by one-way ANOVA followed by Dunnett's multiple comparison test; *, p < 0.05 vs. control.

### Detailed Description

The p19 subunit of IL-23 (also referred to herein as "IL-23A", "IL-23p19" and "p19 subunit") is a 189 amino acid polypeptide containing a 21 aa leader sequence (Oppmann et al. Immunity 13:715 (2000), SEQ ID NO: 22). The biological activity of the molecule is only detected when it is partnered with the IL-12p40 subunit to form IL-23. IL-23 is predominantly expressed by activated dendritic cells (DCs) and phagocytic cells.

SEQ ID NO: 22:
mlgsravmll lllpwtaqgr avpggsspaw tqcqqlsqkl ctlawsahpl vghmdlreeg
deettndvph iqcgdgcdpq glrdnsqfcl qrihqglify ekllgsdift gepsllpdsp
vgqlhasllg lsqllqpegh hwetqqipsl spsqpwqrll lrfkilrslq afvavaarvf
ahgaatlsp

The receptor for IL-23 was found to be composed of the IL-12Rβ1 subunit of IL-12 receptor partnered with a unique subunit called IL-23R (Parham et al. J. Immunol. 168:5699 (2002)). Expression of the receptor is detected primarily on memory T cells (e.g. Th17 cells) and NK cells. Thus, expression of this cytokine:receptor pair appears to be restricted to specific populations of immune cells. IL-23 was found to be critically involved in the production and maintenance of Th17 cells (Kikly et al. Curr. Opin. Immunol. 18:670 (2006), Kastelein et al. Ann. Rev. Immunol. 25:221 (2007)). These cells produce IL-17A, IL-17F, IL-22 and other pro-inflammatory cytokines such as IL-6 and TNF-α. Recently, it became apparent that IL23R marks a specific pathogenic subset of Th17 cells which secretes GM-CSF in experimental autoimmune encephalomyelitis, indicating that a specific subset of Th17 cells might drive disease. As described below, animal model studies on the role of these Th17 cells show their importance as a driving force in chronic inflammation and autoimmunity.

More recently, the role of Th17 cells, and also of Th2 cells and Th2/Th17 dual-positive cells, as well as of cytokines expressed or controlled by these types of cells has been investigated in asthma (i.e. IL17A, IL17F). However, it remains unclear whether targeting one of these cytokines, in particular IL-23, could lead to a therapeutic benefits to severe asthma patients, for example by reduction of exacerbations.

The present invention provides methods for treating asthma, in particular severe persistent asthma, comprising administering an anti-IL-23A antibody to a patient. In one aspect, a method of the present invention is for the treatment of patients suffering from neutrophilic asthma, i.e. patients having elevated levels of neutrophils in the sputum, also called neutrophilic asthma patients. Accordingly, in one aspect, in a method of the present invention a patient is a neutrophilic asthma patient. In another aspect, a method of the present invention is for the treatment of patients suffering from eosinophilic asthma, i.e. patients having elevated levels of eosinophils in the sputum, also called eosinophilic asthma patients. Accordingly, in one aspect, in a method of the present invention a patient is a eosinophilic asthma patient. In another aspect, a method of the present invention is for the treatment of patients suffering from mixed asthma, i.e. patients having elevated levels of neutrophils and eosinophils in the sputum. Accordingly, in one aspect, in a method of the present invention a patient is a patient with mixed asthma. In another aspect, a method of the present invention is for the treatment of patients suffering from paucigranulocytic asthma, i.e. patients having normal or below normal levels of granulocytes in the sputum. Accordingly, in one aspect, in a method of the present invention a patient is a patient with paucigranulocytic asthma.

In one aspect, severe persistent asthma is referred to as in the previous classifications of the GINA guidelines (Global Initiative for Asthma), for example the 2012 and 2014 classifications, but the inclusion and exclusion criteria for asthma patients are defined in accordance with the most recent GINA guidelines regarding control of asthma (GINA 2015). These are patients that require Step 4 or 5 treatment regimens per the 2015 guidelines (GINA 2015).

In one aspect, a patient with severe persistent asthma is also taking medium or high dose ICS plus at least one controller medication (e.g. LABA, LTRA, theophylline).

In one aspect, the type of asthma is categorized by induced sputum, by blood cell counts or by blood biomarkers which could be eosinophiles and/ or various cytokines or other proteins or gene expression.

In a further aspect, in a method of the present invention a patient is treated for asthma, in particular severe persistent asthma, that is mediated by Th17 cells or cells responsive to IL-23. In a further aspect, in a method of the present invention a patient is treated for asthma, in particular severe persistent asthma, that is mediated by Th2 cells. In a further aspect, in a method of the present invention a patient is treated for asthma, in particular severe persistent asthma, that is mediated by Th2/Th17 dual-positive cells or cells responsive to Th2 responsive cytokines and IL-23.

In a further aspect, the present invention provides a method for the treatment or prophylaxis of asthma exacerbations comprising administering an anti-IL-23A antibody to a patient with severe persistent asthma.

In a further aspect, the present invention provides a method for the treatment or control of symptoms of severe asthma comprising administering an anti-IL-23A antibody to a patient with severe persistent asthma.

In a further aspect, the present invention provides a method for the treatment or prophylaxis of asthma exacerbations and for the treatment or control of symptoms of severe asthma comprising administering an anti-IL-23A antibody to a patient with severe persistent asthma.

In a further aspect, the present invention provides a method to reduce the rate of asthma exacerbations comprising administering an anti-IL-23A antibody to a patient with severe persistent asthma.

In a further aspect, the present invention provides a method for treating severe persistent asthma comprising subcutaneously administering to an adult patient an anti-IL-23A antibody in a schedule and dose for a time sufficient to reduce the symptoms of asthma.

In a further aspect, the present invention provides a method for treating severe persistent asthma comprising subcutaneously administering to an adult patient an anti-IL-23A antibody once every four weeks at a dose of 90 mg, whereby the symptoms of asthma are reduced.

In a further aspect, the present invention provides a method for treating severe persistent asthma comprising subcutaneously administering to an adult patient an anti-IL-23A antibody selected from the group consisting of Antibody A, Antibody B, Antibody C or Antibody D, once every four weeks at a dose of 90 mg, whereby the symptoms of asthma are reduced.

In a further aspect, the present invention provides a method for treating severe persistent asthma comprising subcutaneously administering to an adult patient anti-IL-23A Antibody A once every four weeks at a dose of 90 mg, whereby the symptoms of asthma are reduced.

In one aspect, in a method described herein, the treatment, prophylaxis, control or reduction is measured by the reduction of annual rate of exacerbations in said patient, for example with a hazard ratio (HR) of 0.75.

In one aspect, in a method described herein, the treatment, prophylaxis, control or reduction is measured by the relative improvement in proportion of patients having a Asthma Control Questionnaire (ACQ) reduction of 0.5 units: 20 %.

In one aspect, in a method described herein, the treatment, prophylaxis, control or reduction is measured by improvement in trough FEV₁ (e.g. > 125ml), improvement in health related QoL (e.g. Asthma Quality of Life Questionnaire (AQLQ)), or improvement in symptoms score / rescue medication use.

In a further aspect, in a method of the present invention, the patient is not adequately controlled on inhaled corticosteroids. In a further aspect, the patient is not adequately controlled on inhaled corticosteroids and a second controller medication. In a further aspect, the patient is 12 years of age and older.

In one aspect, the present invention provides an anti-IL-23A antibody for use in a method described herein, in particular in the treament of asthma, in particular severe persistent asthma, for example as described herein.

In one aspect, the present invention provides for the use of an anti-IL-23A antibody for the preparation of a medicament for the treatment of asthma, in particular severe persistent asthma, for example as described herein.

In one embodiment, an anti-IL-23A antibody in any one of the methods above is disclosed herein.

In one aspect, in any one of the methods above, a pharmaceutical composition comprising an anti-IL-23A antibody is administered to the patient. In one aspect, formulation 2 disclosed in Example 3 comprising an anti-IL-23A antibody, for example Antibody A, Antibody B, Antibody C or Antibody D is administered to the patient. In one aspect, formulation 3 disclosed in Example 3 comprising an anti-IL-23A antibody, for example Antibody A, Antibody B, Antibody C or Antibody D is administered to the patient.

In one aspect, the anti-IL-23A antibody is a humanized antibody. In one aspect, the anti-IL-23A antibody is a monoclonal antibody. In one aspect, the anti-IL-23A antibody is a full length antibody. In one aspect, the anti-IL-23A antibody is a humanized monoclonal antibody, for example a full length humanized monoclonal antibody.

An antibody described herein recognizes specific "IL-23A antigen epitope" or" IL-23A epitope". As used herein these terms refer to a molecule (e.g., a peptide) or a fragment of a molecule capable of immunoreactivity with an anti-IL-23A antibody and, for example, include an IL-23A antigenic determinant recognized by any of the antibodies having a light chain/heavy chain sequence combination of SEQ ID NO:11/14, 11/15, 10/14 or 10/15.

The generalized structure of antibodies or immunoglobulin is well known to those of skill in the art. These molecules are heterotetrameric glycoproteins, typically of about 150,000 daltons, composed of two identical light (L) chains and two identical heavy (H) chains and are typically referred to as full length antibodies. Each light chain is covalently linked to a heavy chain by one disulfide bond to form a heterodimer, and the heterotrameric molecule is formed through a covalent disulfide linkage between the two identical heavy chains of the heterodimers. Although the light and heavy chains are linked together by one disulfide bond, the number of disulfide linkages between the two heavy chains varies by immunoglobulin isotype. Each heavy and light chain also has regularly spaced intrachain disulfide bridges. Each heavy chain has at the amino-terminus a variable domain (V_{H}), followed by three or four constant domains (C_{H1}, C_{H2}, C_{H3}, and C_{H4}), as well as a hinge region between C_{H1} and C_{H2}. Each light chain has two domains, an amino-terminal variable domain (V_{L}) and a carboxy-terminal constant domain (C_{L}). The V_{L} domain associates non-covalently with the V_{H} domain, whereas the C_{L} domain is commonly covalently linked to the C_{H1} domain via a disulfide bond. Particular amino acid residues are believed to form an interface between the light and heavy chain variable domains (Chothia et al., 1985, J. Mol. Biol. 186:651-663). Variable domains are also referred herein as variable regions.

Certain domains within the variable domains differ extensively between different antibodies i.e., are "hypervariable." These hypervariable domains contain residues that are directly involved in the binding and specificity of each particular antibody for its specific antigenic determinant. Hypervariability, both in the light chain and the heavy chain variable domains, is concentrated in three segments known as complementarity determining regions (CDRs) or hypervariable loops (HVLs). CDRs are defined by sequence comparison in Kabat et al., 1991, In: Sequences of Proteins of Immunological Interest, 5th Ed. Public Health Service, National Institutes of Health, Bethesda, Md., whereas HVLs (also referred herein as CDRs) are structurally defined according to the three-dimensional structure of the variable domain, as described by Chothia and Lesk, 1987, J. Mol. Biol. 196: 901-917. These two methods result in slightly different identifications of a CDR. As defined by Kabat, CDR-L1 is positioned at about residues 24-34, CDR-L2, at about residues 50-56, and CDR-L3, at about residues 89-97 in the light chain variable domain; CDR-H1 is positioned at about residues 31-35, CDR-H2 at about residues 50-65, and CDR-H3 at about residues 95-102 in the heavy chain variable domain. The exact residue numbers that encompass a particular CDR will vary depending on the sequence and size of the CDR. Those skilled in the art can routinely determine which residues comprise a particular CDR given the variable region amino acid sequence of the antibody. The CDR1, CDR2, CDR3 of the heavy and light chains therefore define the unique and functional properties specific for a given antibody.

The three CDRs within each of the heavy and light chains are separated by framework regions (FR), which contain sequences that tend to be less variable. From the amino terminus to the carboxy terminus of the heavy and light chain variable domains, the FRs and CDRs are arranged in the order: FR1, CDR1, FR2, CDR2, FR3, CDR3, and FR4. The largely β-sheet configuration of the FRs brings the CDRs within each of the chains into close proximity to each other as well as to the CDRs from the other chain. The resulting conformation contributes to the antigen binding site (see Kabat et al., 1991, NIH Publ. No. 91-3242, Vol. I, pages 647-669), although not all CDR residues are necessarily directly involved in antigen binding.

FR residues and Ig constant domains are not directly involved in antigen binding, but contribute to antigen binding and/or mediate antibody effector function. Some FR residues are thought to have a significant effect on antigen binding in at least three ways: by noncovalently binding directly to an epitope, by interacting with one or more CDR residues, and by affecting the interface between the heavy and light chains. The constant domains are not directly involved in antigen binding but mediate various Ig effector functions, such as participation of the antibody in antibody dependent cellular cytotoxicity (ADCC), complement dependent cytotoxicity (CDC) and antibody dependent cellular phagocytosis (ADCP).

The light chains of vertebrate immunoglobulins are assigned to one of two clearly distinct classes, kappa (κ) and lambda (λ), based on the amino acid sequence of the constant domain. By comparison, the heavy chains of mammalian immunoglobulins are assigned to one of five major classes, according to the sequence of the constant domains: IgA, IgD, IgE, IgG, and IgM. IgG and IgA are further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively. The subunit structures and three-dimensional configurations of the classes of native immunoglobulins are well known.

The terms, "antibody", "anti-IL-23A antibody", "anti-IL-23p19 antibody", "humanized anti-IL-23A antibody", "humanized anti-IL-23p19 antibody", "humanized anti-IL-23A epitope antibody", humanized anti-IL-2319 epitope antibody", "variant humanized anti-IL-23A epitope antibody" and "variant humanized anti-IL-23p19 epitope antibody" specifically encompass monoclonal antibodies (including full length monoclonal antibodies), polyclonal antibodies, and antibody fragments such as variable domains and other portions of antibodies that exhibit a desired biological activity, e.g., IL-23A binding. The term "monoclonal antibody" (mAb) refers to an antibody that is highly specific, being directed against a single antigenic determinant, an "epitope". Therefore, the modifier "monoclonal" is indicative of antibodies directed to the identical epitope and is not to be construed as requiring production of the antibody by any particular method. It should be understood that monoclonal antibodies can be made by any technique or methodology known in the art; including e.g., the hybridoma method (Kohler et al., 1975, Nature 256:495), or recombinant DNA methods known in the art (see, e.g., U.S. Pat. No. 4,816,567), or methods of isolation of monoclonal recombinantly produced using phage antibody libraries, using techniques described in Clackson et al., 1991, Nature 352: 624-628, and Marks et al., 1991, J. Mol. Biol. 222: 581-597.

The term "monomer" refers to a homogenous form of an antibody. For example, for a full-length antibody, monomer means a monomeric antibody having two identical heavy chains and two identical light chains.

Chimeric antibodies consist of the heavy and light chain variable regions of an antibody from one species (e.g., a non-human mammal such as a mouse) and the heavy and light chain constant regions of another species (e.g., human) antibody and can be obtained by linking the DNA sequences encoding the variable regions of the antibody from the first species (e.g., mouse) to the DNA sequences for the constant regions of the antibody from the second (e.g. human) species and transforming a host with an expression vector containing the linked sequences to allow it to produce a chimeric antibody. Alternatively, the chimeric antibody also could be one in which one or more regions or domains of the heavy and/or light chain is identical with, homologous to, or a variant of the corresponding sequence in a monoclonal antibody from another immunoglobulin class or isotype, or from a consensus or germline sequence. Chimeric antibodies can include fragments of such antibodies, provided that the antibody fragment exhibits the desired biological activity of its parent antibody, for example binding to the same epitope (see, e.g., U.S. Pat. No. 4,816,567; and Morrison et al., 1984, Proc. Natl. Acad. Sci. USA 81: 6851-6855).

The terms, "antibody fragment", "anti-IL-23A antibody fragment", "anti-IL-23A epitope antibody fragment", "humanized anti-IL-23A antibody fragment", "humanized anti-IL-23A epitope antibody fragment", "variant humanized anti-IL-23A epitope antibody fragment" refer to a portion of a full length anti-IL-23A antibody, in which a variable region or a functional capability is retained, for example, specific IL-23A epitope binding. Examples of antibody fragments include, but are not limited to, a Fab, Fab', F(ab')₂, Fd, Fv, scFv and scFv-Fc fragment.

Full length antibodies can be treated with enzymes such as papain or pepsin to generate useful antibody fragments. Papain digestion is used to produces two identical antigen-binding antibody fragments called "Fab" fragments, each with a single antigen-binding site, and a residual "Fc" fragment. The Fab fragment also contains the constant domain of the light chain and the C_{H1} domain of the heavy chain. Pepsin treatment yields a F(ab')₂ fragment that has two antigen-binding sites and is still capable of crosslinking antigen.

Fab' fragments differ from Fab fragments by the presence of additional residues including one or more cysteines from the antibody hinge region at the C-terminus of the C_{H1} domain. F(ab')₂ antibody fragments are pairs of Fab' fragments linked by cysteine residues in the hinge region. Other chemical couplings of antibody fragments are also known.

"Fv" fragment contains a complete antigen-recognition and binding site consisting of a dimer of one heavy and one light chain variable domain in tight, non-covalent association. In this configuration, the three CDRs of each variable domain interact to define an antigen-biding site on the surface of the V_{H}-V_{L} dimer. Collectively, the six CDRs confer antigen-binding specificity to the antibody.

A "single-chain Fv" or "scFv" antibody fragment is a single chain Fv variant comprising the V_{H} and V_{L} domains of an antibody where the domains are present in a single polypeptide chain. The single chain Fv is capable of recognizing and binding antigen. The scFv polypeptide may optionally also contain a polypeptide linker positioned between the V_{H} and V_{L} domains in order to facilitate formation of a desired three-dimensional structure for antigen binding by the scFv (see, e.g., Pluckthun, 1994, In The Pharmacology of monoclonal Antibodies, Vol. 113, Rosenburg and Moore eds., Springer-Verlag, New York, pp. 269-315).

A "humanized antibody" or a "humanized antibody fragment" is a specific type of chimeric antibody which includes an immunoglobulin amino acid sequence variant, or fragment thereof, which is capable of binding to a predetermined antigen and which, comprises one or more FRs having substantially the amino acid sequence of a human immunoglobulin and one or more CDRs having substantially the amino acid sequence of a non-human immunoglobulin. This non-human amino acid sequence often referred to as an "import" sequence is typically taken from an "import" antibody domain, particularly a variable domain. In general, a humanized antibody includes at least the CDRs or HVLs of a non-human antibody, inserted between the FRs of a human heavy or light chain variable domain. The present invention describes specific humanized anti-IL-23A antibodies which contain CDRs derived from the mouse monoclonal antibodies or humanized CDRs shown in Tables 1 and 2 inserted between the FRs of human germline sequence heavy and light chain variable domains. It will be understood that certain mouse FR residues may be important to the function of the humanized antibodies and therefore certain of the human germline sequence heavy and light chain variable domains residues are modified to be the same as those of the corresponding mouse sequence.

In another aspect, a humanized anti-IL-23A antibody comprises substantially all of at least one, and typically two, variable domains (such as contained, for example, in Fab, Fab', F(ab')2, Fabc, and Fv fragments) in which all, or substantially all, of the CDRs correspond to those of a non-human immunoglobulin, and specifically herein, all of the CDRs are mouse or humanized sequences as detailed in Tables 1 and 2 herein below and all, or substantially all, of the FRs are those of a human immunoglobulin consensus or germline sequence. In another aspect, a humanized anti- IL-23A antibody also includes at least a portion of an immunoglobulin Fc region, typically that of a human immunoglobulin. Ordinarily, the antibody will contain both the light chain as well as at least the variable domain of a heavy chain. The antibody also may include one or more of the C_{H1}, hinge, C_{H2}, C_{H3}, and/or C_{H4} regions of the heavy chain, as appropriate.

A humanized anti-IL-23A antibody can be selected from any class of immunoglobulins, including IgM, IgG, IgD, IgA and IgE, and any isotype, including IgG₁, IgG₂, IgG₃, IgG₄, IgA₁ and IgA₂. For example, the constant domain can be a complement fixing constant domain where it is desired that the humanized antibody exhibit cytotoxic activity, and the isotype is typically IgG₁. Where such cytotoxic activity is not desirable, the constant domain may be of another isotype, e.g., IgG₂. An alternative humanized anti-IL-23A antibody can comprise sequences from more than one immunoglobulin class or isotype, and selecting particular constant domains to optimize desired effector functions is within the ordinary skill in the art. In specific embodiments, the present invention provides antibodies that are IgG1 antibodies and more particularly, are IgG1 antibodies in which there is a knock-out of effector functions.

The FRs and CDRs, or HVLs, of a humanized anti-IL-23A antibody need not correspond precisely to the parental sequences. For example, one or more residues in the import CDR, or HVL, or the consensus or germline FR sequence may be altered (e.g., mutagenized) by substitution, insertion or deletion such that the resulting amino acid residue is no longer identical to the original residue in the corresponding position in either parental sequence but the antibody nevertheless retains the function of binding to IL-23A. Such alteration typically will not be extensive and will be conservative alterations. Usually, at least 75% of the humanized antibody residues will correspond to those of the parental consensus or germline FR and import CDR sequences, more often at least 90%, and most frequently greater than 95%, or greater than 98% or greater than 99%.

Immunoglobulin residues that affect the interface between heavy and light chain variable regions ("the V_{L}-V_{H} interface") are those that affect the proximity or orientation of the two chains with respect to one another. Certain residues that may be involved in interchain interactions include V_{L} residues 34, 36, 38, 44, 46, 87, 89, 91, 96, and 98 and V_{H} residues 35, 37, 39, 45, 47, 91, 93, 95, 100, and 103 (utilizing the numbering system set forth in Kabat et al., Sequences of Proteins of Immunological Interest (National Institutes of Health, Bethesda, Md., 1987)). U.S. Pat. No. 6,407,213 also discusses that residues such as V_{L} residues 43 and 85, and V_{H} residues 43 and 60 also may be involved in this interaction. While these residues are indicated for human IgG only, they are applicable across species. Important antibody residues that are reasonably expected to be involved in interchain interactions are selected for substitution into the consensus sequence.

The terms "consensus sequence" and "consensus antibody" refer to an amino acid sequence which comprises the most frequently occurring amino acid residue at each location in all immunoglobulins of any particular class, isotype, or subunit structure, e.g., a human immunoglobulin variable domain. The consensus sequence may be based on immunoglobulins of a particular species or of many species. A "consensus" sequence, structure, or antibody is understood to encompass a consensus human sequence as described in certain embodiments, and to refer to an amino acid sequence which comprises the most frequently occurring amino acid residues at each location in all human immunoglobulins of any particular class, isotype, or subunit structure. Thus, the consensus sequence contains an amino acid sequence having at each position an amino acid that is present in one or more known immunoglobulins, but which may not exactly duplicate the entire amino acid sequence of any single immunoglobulin. The variable region consensus sequence is not obtained from any naturally produced antibody or immunoglobulin. Kabat et al., 1991, Sequences of Proteins of Immunological Interest, 5th Ed. Public Health Service, National Institutes of Health, Bethesda, Md., and variants thereof. The FRs of heavy and light chain consensus sequences, and variants thereof, provide useful sequences for the preparation of humanized anti-IL-23p19 antibodies. See, for example, U.S. Pat. Nos. 6,037,454 and 6,054,297.

Human germline sequences are found naturally in the human population. A combination of those germline genes generates antibody diversity. Germline antibody sequences for the light chain of the antibody come from conserved human germline kappa or lambda v-genes and j-genes. Similarly the heavy chain sequences come from germline v-, d- and j-genes (LeFranc, M-P, and LeFranc, G, "The Immunoglobulin Facts Book" Academic Press, 2001).

As used herein, "variant", "anti- IL-23A variant", "humanized anti- IL-23A variant", or "variant humanized anti- IL-23A" each refers to a humanized anti-IL-23A antibody having at least a light chain variable murine CDR from any of the sequences as shown in Table 1 or a heavy chain murine CDR sequence derived from the murine monoclonal antibody as shown in Table 2. Variants include those having one or more amino acid changes in one or both light chain or heavy chain variable domains, provided that the amino acid change does not substantially impair binding of the antibody to IL-23A. Exemplary antibodies produced herein include those designated as Antibody A, Antibody B, Antibody C and Antibody D, and the various light chains and heavy chains of the same are shown in SEQ ID Nos:18 and 21, and SEQ ID Nos:19 and 20, respectively.

An "isolated" antibody is one that has been identified and separated and/or recovered from a component of its natural environment. Contaminant components of the antibody's natural environment are those materials that may interfere with diagnostic or therapeutic uses of the antibody, and can be enzymes, hormones, or other proteinaceous or nonproteinaceous solutes. In one aspect, the antibody will be purified to at least greater than 95% isolation by weight of antibody.

An isolated antibody includes an antibody in situ within recombinant cells in which it is produced, since at least one component of the antibody's natural environment will not be present. Ordinarily however, an isolated antibody will be prepared by at least one purification step in which the recombinant cellular material is removed.

The term "antibody performance" refers to factors that contribute to antibody recognition of antigen or the effectiveness of an antibody in vivo. Changes in the amino acid sequence of an antibody can affect antibody properties such as folding, and can influence physical factors such as initial rate of antibody binding to antigen (kₐ), dissociation constant of the antibody from antigen (k_{d}), affinity constant of the antibody for the antigen (Kd), conformation of the antibody, protein stability, and half life of the antibody.

The term "epitope tagged" when used herein, refers to an anti-IL-23A antibody fused to an "epitope tag". An "epitope tag" is a polypeptide having a sufficient number of amino acids to provide an epitope for antibody production, yet is designed such that it does not interfere with the desired activity of the anti-IL-23A antibody. The epitope tag is usually sufficiently unique such that an antibody raised against the epitope tag does not substantially cross-react with other epitopes. Suitable tag polypeptides generally contain at least 6 amino acid residues and usually contain about 8 to 50 amino acid residues, or about 9 to 30 residues. Examples of epitope tags and the antibody that binds the epitope include the flu HA tag polypeptide and its antibody 12CA5 (Field et al., 1988 Mol. Cell. Biol. 8: 2159-2165; c-myc tag and 8F9, 3C7, 6E10, G4, B7 and 9E10 antibodies thereto (Evan et al., 1985, Mol. Cell. Biol. 5(12):3610-3616; and Herpes simplex virus glycoprotein D (gD) tag and its antibody (Paborsky et al. 1990, Protein Engineering 3(6): 547-553). In certain embodiments, the epitope tag is a "salvage receptor binding epitope". As used herein, the term "salvage receptor binding epitope" refers to an epitope of the Fc region of an IgG molecule (such as IgG₁, IgG₂, IgG₃, or IgG₄) that is responsible for increasing the in vivo serum half-life of the IgG molecule.

For diagnostic as well as therapeutic monitoring purposes, the antibodies of the invention also may be conjugated to a label, either a label alone or a label and an additional second agent (prodrug, chemotherapeutic agent and the like). A label, as distinguished from the other second agents refers to an agent that is a detectable compound or composition and it may be conjugated directly or indirectly to a antibody of the present invention. The label may itself be detectable (e.g., radioisotope labels or fluorescent labels) or, in the case of an enzymatic label, may catalyze chemical alteration of a substrate compound or composition that is detectable. Labeled anti-IL-23A antibody can be prepared and used in various applications including in vitro and in vivo diagnostics.

In various aspects of the present invention one or more domains of the antibodies will be recombinantly expressed. Such recombinant expression may employ one or more control sequences, i.e., polynucleotide sequences necessary for expression of an operably linked coding sequence in a particular host organism. The control sequences suitable for use in prokaryotic cells include, for example, promoter, operator, and ribosome binding site sequences. Eukaryotic control sequences include, but are not limited to, promoters, polyadenylation signals, and enhancers. These control sequences can be utilized for expression and production of anti-IL-23A antibody in prokaryotic and eukaryotic host cells.

A nucleic acid sequence is "operably linked" when it is placed into a functional relationship with another nucleic acid sequence. For example, a nucleic acid presequence or secretory leader is operably linked to a nucleic acid encoding a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading frame. However, enhancers are optionally contiguous. Linking can be accomplished by ligation at convenient restriction sites. If such sites do not exist, synthetic oligonucleotide adaptors or linkers can be used.

As used herein, the expressions "cell", "cell line", and "cell culture" are used interchangeably and all such designations include the progeny thereof. Thus, "transformants" and "transformed cells" include the primary subject cell and cultures derived therefrom without regard for the number of transfers.

The term "mammal" for purposes of treatment refers to any animal classified as a mammal, including humans, domesticated and farm animals, and zoo, sports, or pet animals, such as dogs, horses, cats, cows, and the like. Preferably, the mammal is human.

A "disorder", as used herein, is any condition that would benefit from treatment with an anti-IL-23A antibody described herein. This includes chronic and acute disorders or diseases including those pathological conditions that predispose the mammal to the disorder in question.

As used herein, the term "IL-23-associated disorder" or "IL-23-associated disease" refers to a condition in which IL-23 activity contributes to the disease and typically where IL-23 is abnormally expressed. An IL-23-associated disorder includes diseases and disorders of the immune system, such as autoimmune disorders and inflammatory diseases. Such conditions include psoriasis, inflammatory bowel disease, for example ulcerative colitis or Crohn's disease, and spondyloarthritis, for example ankylosing spondylitis, non-radiographic axial spondyloarthritis, peripheral spondyloarthritis or psoriatic arthritis.

The term "intravenous infusion" refers to introduction of an agent into the vein of an animal or human patient over a period of time greater than approximately 15 minutes, generally between approximately 30 to 90 minutes.

The term "intravenous bolus" or "intravenous push" refers to drug administration into a vein of an animal or human such that the body receives the drug in approximately 15 minutes or less, generally 5 minutes or less.

The term "subcutaneous administration" refers to introduction of an agent under the skin of an animal or human patient, preferable within a pocket between the skin and underlying tissue, by relatively slow, sustained delivery from a drug receptacle. Pinching or drawing the skin up and away from underlying tissue may create the pocket.

The term "subcutaneous infusion" refers to introduction of a drug under the skin of an animal or human patient, preferably within a pocket between the skin and underlying tissue, by relatively slow, sustained delivery from a drug receptacle for a period of time including, but not limited to, 30 minutes or less, or 90 minutes or less. Optionally, the infusion may be made by subcutaneous implantation of a drug delivery pump implanted under the skin of the animal or human patient, wherein the pump delivers a predetermined amount of drug for a predetermined period of time, such as 30 minutes, 90 minutes, or a time period spanning the length of the treatment regimen.

The term "subcutaneous bolus" refers to drug administration beneath the skin of an animal or human patient, where bolus drug delivery is less than approximately 15 minutes; in another aspect, less than 5 minutes, and in still another aspect, less than 60 seconds. In yet even another aspect, administration is within a pocket between the skin and underlying tissue, where the pocket may be created by pinching or drawing the skin up and away from underlying tissue.

The term "therapeutically effective amount" is used to refer to an amount of an active agent that relieves or ameliorates one or more of the symptoms of the disorder being treated. In another aspect, the therapeutically effective amount refers to a target serum concentration that has been shown to be effective in, for example, slowing disease progression. Efficacy can be measured in conventional ways, depending on the condition to be treated.

The terms "treatment" and "therapy" and the like, as used herein, are meant to include therapeutic as well as prophylactic, or suppressive measures for a disease or disorder leading to any clinically desirable or beneficial effect, including but not limited to alleviation or relief of one or more symptoms, regression, slowing or cessation of progression of the disease or disorder. Thus, for example, the term treatment includes the administration of an agent prior to or following the onset of a symptom of a disease or disorder thereby preventing or removing one or more signs of the disease or disorder. As another example, the term includes the administration of an agent after clinical manifestation of the disease to combat the symptoms of the disease. Further, administration of an agent after onset and after clinical symptoms have developed where administration affects clinical parameters of the disease or disorder, such as the degree of tissue injury or the amount or extent of metastasis, whether or not the treatment leads to amelioration of the disease, comprises "treatment" or "therapy" as used herein. Moreover, as long as the compositions of the invention either alone or in combination with another therapeutic agent alleviate or ameliorate at least one symptom of a disorder being treated as compared to that symptom in the absence of use of the anti-IL-23A antibody composition, the result should be considered an effective treatment of the underlying disorder regardless of whether all the symptoms of the disorder are alleviated or not.

The term "package insert" is used to refer to instructions customarily included in commercial packages of therapeutic products, that contain information about the indications, usage, administration, contraindications and/or warnings concerning the use of such therapeutic products.

### Antibodies

The CDRs of selected antibodies used in the context of the present invention are shown in Table 1 and 2. The variable regions of selected antibodies used in the context of the present invention are shown in Table 3 and 4.

**Table 1: LIGHT CHAIN CDR sequences**

| | L-CDR1 | L-CDR2 | L-CDR3 |
|---|---|---|---|
| 6B8 | KASRDVAIAVA (SEQ ID NO:1) | WASTRHT (SEQ ID NO:2) | HQYSSYPFT (SEQ ID NO:3) |

**Table 2: HEAVY CHAIN CDR sequences**

| | H-CDR1 | H-CDR2 | H-CDR3 |
|---|---|---|---|
| 6B8 | GNTFTDQTIH (SEQ ID NO:4) | YIYPRDDSPKYNENFKG (SEQ ID NO:5) | PDRSGYAWFIY (SEQ ID NO:6) |
| Hu_6B8-2 | GYTFTDQTIH (SEQ ID NO:7) | YIYPRDDSPKYNENFKG (SEQ ID NO:5) | PDRSGYAWFIY (SEQ ID NO:6) |
| Hu_6B8-5 | GFTFTDQTIH (SEQ ID NO:8) | YIYPRDDSPKYNENFKG (SEQ ID NO:5) | PDRSGYAWFIY (SEQ ID NO:6) |
| Hu_6B8-36/65 | GGTFTDQTIH (SEQ ID NO:9) | YIYPRDDSPKYNENFKG (SEQ ID NO:5) | PDRSGYAWFIY (SEQ ID NO:6) |

**Table 3: Humanized 6B8-VK Sequences**

| | |
|---|---|
| 6B8CVK-65 | DIQMTQSPSSLSASVGDRVTITCKASRDVAIAVAWYQQKPGKVPKLL LFWASTRHTGVPDRFSGSGSGTDFTLTISSLQPEDLADYYCHQYSSY PFTFGQGTKLEIK (SEQ ID NO:10) |
| 6B8CVK-66 | DIQMTQSPSSLSASVGDRVTITCKASRDVAIAVAWYQQKPGKVPKLL IYWASTRHTGVPSRFSGSGSRTDFTLTISSLQPEDVADYFCHQYSSY PFTFGSGTKLEIK (SEQ ID NO:11) |
| 6B8CVK-67 | DIQMTQSPSSLSASVGDRVTITCKASRDVAIAVAWYQQKPGKVPKLL LYWASTRHTGVPSRFSGSGSRTDFTLTISSLQPEDVATYYCHQYSSY PFTFGSGTKLEIK (SEQ ID NO:12) |
| 6B8CVK-78 | DIQMTQSPSSLSASVGDRVTITCKASRDVAIAVAWYQQKPGKVPKLL LFWASTRHTGVPDRFSGSGSRTDFTLTISSLQPEDLADYYCHQYSSY PFTFGSGTKLEIK (SEQ ID NO:13) |

**Table 4: Humanized 6B8-VH Sequence**

| | |
|---|---|
| 6B8CVH-02 | QVQLVQSGAEVKKPGSSVKVSCKASGYTFTDQTIHWMRQAPGQGLEW IGYIYPRDDSPKYNENFKGKVTITADKSTSTAYMELSSLRSEDTAVY YCAIPDRSGYAWFIYWGQGTLVTVSS (SEQ ID NO:14) |
| 6B8CVH-05 | QVQLVQSGAEVKKPGSSVKVSCKASGFTFTDQTIHWVRQAPGQGLEW MGYIYPRDDSPKYNENFKGKVTLTADKSTSTAYMELSSLRSEDTAVY YCAIPDRSGYAWFIYWGQGTLVTVSS (SEQ ID NO:15) |
| 6B8CVH-36 | QVQLVQSGAEVKKPGSSVKTSCKASGGTFTDQTIHWVRQRPGQGLEW MGYIYPRDDSPKYNENFKGRVTITADKSTSTAYMELSSLRSEDTAVY YCAIPDRSGYAWFIYWGQGTLVTVSS (SEQ ID NO:16) |
| 6B8CVH-65 | QVQLVQSGAEVKKPGSSVKVSCKASGGTFTDQTIHWVRQAPGQGLEW MGYIYPRDDSPKYNENFKGRVTLTADKSTSTAYMELSSLRSEDTAVY FCARPDRSGYAWFIYWGQGTLVTVSS (SEQ ID NO:17) |

Selected combination of humanized light chain and heavy chain variable regions derived from mouse antibody 6B8 resulted in Antibodies A, B, C and D:
Antibody A: 6B8-IgG1KO-2 with IgK-66 (heavy chain variable region 6B8CVH-02 and light chain variable region 6B8CVK-66);
Antibody B: 6B8-IgG1KO-5 with IgK-66 (heavy chain variable region 6B8CVH-05 and light chain variable region 6B8CVK-66);
Antibody C: 6B8-IgG1KO-2 with IgK-65 (heavy chain variable region 6B8CVH-02 and light chain variable region 6B8CVK-65);
Antibody D: 6B8-IgG1KO-5 with IgK-65 (heavy chain variable region 6B8CVH-05 and light chain variable region 6B8CVK-65).

Antibodies A, B, C and D have the heavy and light chain sequences shown in Table 5.

**Table 5: Heavy and Light Chain DNA and Amino Acid Sequences for Antibodies A, B, C, and D**

| | | |
|---|---|---|
| **Antibody A** | IgK light Chain #66 | DIQMTQSPSSLSASVGDRVTITCKASRDVAIAVAWYQ QKPGKVPKLLIYWASTRHTGVPSRFSGSGSRTDFTLT ISSLQPEDVADYFCHQYSSYPFTFGSGTKLEIKRTVA APSVFIFPPSDEQLKSGTASVVCLLNNFYPREAKVQW KVDNALQSGNSQESVTEQDSKDSTYSLSSTLTLSKAD YEKHKVYACEVTHQGLSSPVTKSFNRGEC (SEQ ID NO:18) |
| | IgG1 KO Heavy Chain #2 | QVQLVQSGAEVKKPGSSVKVSCKASGYTFTDQTIHWM RQAPGQGLEWIGYIYPRDDSPKYNENFKGKVTITADK STSTAYMELSSLRSEDTAVYYCAIPDRSGYAWFIYWG QGTLVTVSSASTKGPSVFPLAPSSKSTSGGTAALGCL VKDYFPEPVTVSWNSGALTSGVHTFPAVLQSSGLYSL SSVVTVPSSSLGTQTYICNVNHKPSNTKVDKRVEPKS CDKTHTCPPCPAPEAAGGPSVFLFPPKPKDTLMISRT PEVTCVVVDVSHEDPEVKFNWYVDGVEVHNAKTKPRE EQYNSTYRVVSVLTVLHQDWLNGKEYKCKVSNKALPA PIEKTISKAKGQPREPQVYTLPPSREEMTKNQVSLTC LVKGFYPSDIAVEWESNGQPENNYKTTPPVLDSDGSF FLYSKLTVDKSRWQQGNVFSCSVMHEALHNHYTQKSL SLSPG (SEQ ID NO:19) |
| | | |
| **Antibody B** | IgK light Chain #66 | (SEQ ID NO:18) |
| | IgG1KO Heavy Chain | QVQLVQSGAEVKKPGSSVKVSCKASGFTFTDQTIHWV RQAPGQGLEWMGYIYPRDDSPKYNENFKGKVTLTADK STSTAYMELSSLRSEDTAVYYCAIPDRSGYAWFIYWG |
| | #5 | QGTLVTVSSASTKGPSVFPLAPSSKSTSGGTAALGCL VKDYFPEPVTVSWNSGALTSGVHTFPAVLQSSGLYSL SSVVTVPSSSLGTQTYICNVNHKPSNTKVDKRVEPKS CDKTHTCPPCPAPEAAGGPSVFLFPPKPKDTLMISRT PEVTCVVVDVSHEDPEVKFNWYVDGVEVHNAKTKPRE EQYNSTYRVVSVLTVLHQDWLNGKEYKCKVSNKALPA PIEKTISKAKGQPREPQVYTLPPSREEMTKNQVSLTC LVKGFYPSDIAVEWESNGQPENNYKTTPPVLDSDGSF FLYSKLTVDKSRWQQGNVFSCSVMHEALHNHYTQKSL SLSPG (SEQ ID NO:20) |
| | | |
| **Antibody C** | IgK light Chain #65 | DIQMTQSPSSLSASVGDRVTITCKASRDVAIAVAWYQ QKPGKVPKLLLFWASTRHTGVPDRFSGSGSGTDFTLT ISSLQPEDLADYYCHQYSSYPFTFGQGTKLEIKRTVA APSVFIFPPSDEQLKSGTASVVCLLNNFYPREAKVQW KVDNALQSGNSQESVTEQDSKDSTYSLSSTLTLSKAD YEKHKVYACEVTHQGLSSPVTKSFNRGEC (SEQ ID NO:21) |
| | IgG1KO Heavy Chain #2 | (SEQ ID NO:19) |
| | | |
| **Antibody D** | IgK light Chain #65 | (SEQ ID NO:21) |
| | IgG1KO Heavy Chain | (SEQ ID NO:20) |
| | #5 | |

Light chains and heavy chain variable regions of Antibodies A, B, C, and D are underlined in Table 5 above.

In one embodiment, an anti-IL-23A antibody comprises the light chain sequence of SEQ ID NO:18 and the heavy chain sequence of SEQ ID NO:19. In one embodiment, an anti-IL-23A antibody comprises the light chain sequence of SEQ ID NO:18 and the heavy chain sequence of SEQ ID NO:20. In one embodiment, an anti-IL-23A antibody comprises the light chain sequence of SEQ ID NO:21 and the heavy chain sequence of SEQ ID NO:19. In one embodiment, an anti-IL-23A antibody comprises the light chain sequence of SEQ ID NO:21 and the heavy chain sequence of SEQ ID NO:20.

In one embodiment, an anti-IL-23A antibody consists of the light chain sequence of SEQ ID NO:18 and the heavy chain sequence of SEQ ID NO:19. In one embodiment, an anti-IL-23A antibody consists of the light chain sequence of SEQ ID NO:18 and the heavy chain sequence of SEQ ID NO:20. In one embodiment, an anti-IL-23A antibody consists of the light chain sequence of SEQ ID NO:21 and the heavy chain sequence of SEQ ID NO:19. In one embodiment, an anti-IL-23A antibody consists of the light chain sequence of SEQ ID NO:21 and the heavy chain sequence of SEQ ID NO:20.

In a further embodiment, an anti-IL-23A antibody binds to human IL-23A at an epitope consisting of amino acid residues 108 to 126 and amino acid residues 137 to 151 of SEQ ID NO: 22.

In a further embodiment, an anti-IL-23A antibody competitively binds to human IL-23A with an antibody of the present invention, for example Antibody A, Antibody B, Antibody C or Antibody D described herein. The ability of an antibody to competitively bind to IL-23A can be measured using competitive binding assays known in the art.

In some embodiments, an anti-IL-23A antibody comprises light chain variable region sequences having the amino acid sequence set forth in of SEQ ID NO:10, 11, 12 or 13. In some embodiments, an anti-IL-23A antibody comprises heavy chain variable region sequences having the amino acid sequence set forth in of SEQ ID NO:14, 15, 16 or 17 (see Tables 3 and 4 above). The CDR sequences of these antibodies are shown in Tables 1 and 2. For example, anti-IL-23A antibodies are monoclonal antibodies with the combinations of light chain variable and heavy chain variable regions of SEQ ID NO: 11/14, 11/15, 10/14 or 10/15. Such variable regions can be combined with human constant regions.

### Polynucleotides, Vectors, Host Cells, and Recombinant Methods

Other embodiments encompass isolated polynucleotides that comprise a sequence encoding an anti-IL-23A antibody, vectors, and host cells comprising the polynucleotides, and recombinant techniques for production of the humanized antibody. The isolated polynucleotides can encode any desired form of the anti-IL-23A antibody including, for example, full length monoclonal antibodies, Fab, Fab', F(ab')₂, and Fv fragments.

The polynucleotide(s) that comprise a sequence encoding an anti-IL-23A antibody can be fused to one or more regulatory or control sequence, as known in the art, and can be contained in suitable expression vectors or host cell as known in the art. Each of the polynucleotide molecules encoding the heavy or light chain variable domains can be independently fused to a polynucleotide sequence encoding a constant domain, such as a human constant domain, enabling the production of intact antibodies. Alternatively, polynucleotides, or portions thereof, can be fused together, providing a template for production of a single chain antibody.

For recombinant production, a polynucleotide encoding the antibody is inserted into a replicable vector for cloning (amplification of the DNA) or for expression. Many suitable vectors for expressing the recombinant antibody are available. The vector components generally include, but are not limited to, one or more of the following: a signal sequence, an origin of replication, one or more marker genes, an enhancer element, a promoter, and a transcription termination sequence.

The anti-IL-23A antibodies can also be produced as fusion polypeptides, in which the antibody is fused with a heterologous polypeptide, such as a signal sequence or other polypeptide having a specific cleavage site at the amino terminus of the mature protein or polypeptide. The heterologous signal sequence selected is typically one that is recognized and processed (i.e., cleaved by a signal peptidase) by the host cell. For prokaryotic host cells that do not recognize and process the anti-IL-23A antibody signal sequence, the signal sequence can be substituted by a prokaryotic signal sequence. The signal sequence can be, for example, alkaline phosphatase, penicillinase, lipoprotein, heat-stable enterotoxin II leaders, and the like. For yeast secretion, the native signal sequence can be substituted, for example, with a leader sequence obtained from yeast invertase alpha-factor (including Saccharomyces and Kluyveromyces α-factor leaders), acid phosphatase, C. albicans glucoamylase, or the signal described in WO90/13646. In mammalian cells, mammalian signal sequences as well as viral secretory leaders, for example, the herpes simplex gD signal, can be used. The DNA for such precursor region is ligated in reading frame to DNA encoding the anti-IL-23A antibody.

Expression and cloning vectors contain a nucleic acid sequence that enables the vector to replicate in one or more selected host cells. Generally, in cloning vectors this sequence is one that enables the vector to replicate independently of the host chromosomal DNA, and includes origins of replication or autonomously replicating sequences. Such sequences are well known for a variety of bacteria, yeast, and viruses. The origin of replication from the plasmid pBR322 is suitable for most Gram-negative bacteria, the 2-υ. plasmid origin is suitable for yeast, and various viral origins (SV40, polyoma, adenovirus, VSV, and BPV) are useful for cloning vectors in mammalian cells. Generally, the origin of replication component is not needed for mammalian expression vectors (the SV40 origin may typically be used only because it contains the early promoter).

Expression and cloning vectors may contain a gene that encodes a selectable marker to facilitate identification of expression. Typical selectable marker genes encode proteins that confer resistance to antibiotics or other toxins, e.g., ampicillin, neomycin, methotrexate, or tetracycline, or alternatively, are complement auxotrophic deficiencies, or in other alternatives supply specific nutrients that are not present in complex media, e.g., the gene encoding D-alanine racemase for Bacilli.

One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin, mycophenolic acid, and hygromycin. Common selectable markers for mammalian cells are those that enable the identification of cells competent to take up a nucleic acid encoding an anti-IL-23A antibody, such as DHFR (dihydrofolate reductase), thymidine kinase, metallothionein-I and -II (such as primate metallothionein genes), adenosine deaminase, ornithine decarboxylase, and the like. Cells transformed with the DHFR selection gene are first identified by culturing all of the transformants in a culture medium that contains methotrexate (Mtx), a competitive antagonist of DHFR. An appropriate host cell when wild-type DHFR is employed is the Chinese hamster ovary (CHO) cell line deficient in DHFR activity (e.g., DG44).

Alternatively, host cells (particularly wild-type hosts that contain endogenous DHFR) transformed or co-transformed with DNA sequences encoding an anti-IL-23A antibody, wild-type DHFR protein, and another selectable marker such as aminoglycoside 3'-phosphotransferase (APH), can be selected by cell growth in medium containing a selection agent for the selectable marker such as an aminoglycosidic antibiotic, e.g., kanamycin, neomycin, or G418. See, e.g., U.S. Pat. No. 4,965,199.

Where the recombinant production is performed in a yeast cell as a host cell, the TRP1 gene present in the yeast plasmid YRp7 (Stinchcomb et al., 1979, Nature 282: 39) can be used as a selectable marker. The TRP1 gene provides a selection marker for a mutant strain of yeast lacking the ability to grow in tryptophan, for example, ATCC No. 44076 or PEP4-1 (Jones, 1977, Genetics 85:12). The presence of the trp1 lesion in the yeast host cell genome then provides an effective environment for detecting transformation by growth in the absence of tryptophan. Similarly, Leu2p-deficient yeast strains such as ATCC 20,622 and 38,626 are complemented by known plasmids bearing the LEU2 gene.

In addition, vectors derived from the 1.6 µm circular plasmid pKD1 can be used for transformation of Kluyveromyces yeasts. Alternatively, an expression system for large-scale production of recombinant calf chymosin was reported for K. lactis (Van den Berg, 1990, Bio/Technology 8:135). Stable multi-copy expression vectors for secretion of mature recombinant human serum albumin by industrial strains of Kluyveromyces have also been disclosed (Fleer et al., 1991, Bio/Technology 9:968-975).

Expression and cloning vectors usually contain a promoter that is recognized by the host organism and is operably linked to the nucleic acid molecule encoding an anti-IL-23p19 antibody or polypeptide chain thereof. Promoters suitable for use with prokaryotic hosts include phoA promoter, β-lactamase and lactose promoter systems, alkaline phosphatase, tryptophan (trp) promoter system, and hybrid promoters such as the tac promoter. Other known bacterial promoters are also suitable. Promoters for use in bacterial systems also will contain a Shine-Dalgarno (S.D.) sequence operably linked to the DNA encoding the anti-IL-23A antibody.

Many eukaryotic promoter sequences are known. Virtually all eukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Examples of suitable promoting sequences for use with yeast hosts include the promoters for 3-phosphoglycerate kinase or other glycolytic enzymes, such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Inducible promoters have the additional advantage of transcription controlled by growth conditions. These include yeast promoter regions for alcohol dehydrogenase 2, isocytochrome C, acid phosphatase, derivative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657. Yeast enhancers also are advantageously used with yeast promoters.

Anti-IL-23A antibody transcription from vectors in mammalian host cells is controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, or from heat-shock promoters, provided such promoters are compatible with the host cell systems.

The early and late promoters of the SV40 virus are conveniently obtained as an SV40 restriction fragment that also contains the SV40 viral origin of replication. The immediate early promoter of the human cytomegalovirus is conveniently obtained as a Hindlll E restriction fragment. A system for expressing DNA in mammalian hosts using the bovine papilloma virus as a vector is disclosed in U.S. Pat. No. 4,419,446. A modification of this system is described in U.S. Pat. No. 4,601,978. See also Reyes et al., 1982, Nature 297:598-601, disclosing expression of human p-interferon cDNA in mouse cells under the control of a thymidine kinase promoter from herpes simplex virus. Alternatively, the Rous sarcoma virus long terminal repeat can be used as the promoter.

Another useful element that can be used in a recombinant expression vector is an enhancer sequence, which is used to increase the transcription of a DNA encoding an anti-IL-23A antibody by higher eukaryotes. Many enhancer sequences are now known from mammalian genes (e.g., globin, elastase, albumin, α-fetoprotein, and insulin). Typically, however, an enhancer from a eukaryotic cell virus is used. Examples include the SV40 enhancer on the late side of the replication origin (bp 100-270), the cytomegalovirus early promoter enhancer, the polyoma enhancer on the late side of the replication origin, and adenovirus enhancers. See also Yaniv, 1982, Nature 297:17-18 for a description of enhancing elements for activation of eukaryotic promoters. The enhancer may be spliced into the vector at a position 5' or 3' to the anti-IL-23A antibody-encoding sequence, but is preferably located at a site 5' from the promoter.

Expression vectors used in eukaryotic host cells (yeast, fungi, insect, plant, animal, human, or nucleated cells from other multicellular organisms) can also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding anti-IL-23A antibody. One useful transcription termination component is the bovine growth hormone polyadenylation region. See WO94/11026 and the expression vector disclosed therein. In some embodiments, humanized anti-IL-23p19 antibodies can be expressed using the CHEF system. (See, e.g., U.S. Pat. No. 5,888,809; the disclosure of which is incorporated by reference herein.)

Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, Enterobacteriaceae such as Escherichia, e.g., E. coli, Enterobacter, Erwinia, Klebsiella, Proteus, Salmonella, e.g., Salmonella typhimurium, Serratia, e.g., Serratia marcescans, and Shigella, as well as Bacilli such as B. subtilis and B. licheniformis (e.g., B. licheniformis 41 P disclosed in DD 266,710 published Apr. 12, 1989), Pseudomonas such as P. aeruginosa, and Streptomyces. One preferred E. coli cloning host is E. coli 294 (ATCC 31,446), although other strains such as E. coli B, E. coli X1776 (ATCC 31,537), and E. coli W3110 (ATCC 27,325) are suitable. These examples are illustrative rather than limiting.

In addition to prokaryotes, eukaryotic microbes such as filamentous fungi or yeast are suitable cloning or expression hosts for anti-IL-23A antibody-encoding vectors. Saccharomyces cerevisiae, or common baker's yeast, is the most commonly used among lower eukaryotic host microorganisms. However, a number of other genera, species, and strains are commonly available and useful herein, such as Schizosaccharomyces pombe; Kluyveromyces hosts such as, e.g., K. lactis, K. fragilis (ATCC 12,424), K. bulgaricus (ATCC 16,045), K. wickeramii (ATCC 24,178), K. waltii (ATCC 56,500), K. drosophilarum (ATCC 36,906), K. thermotolerans, and K. marxianus; yarrowia (EP 402,226); Pichia pastors (EP 183,070); Candida; Trichoderma reesia (EP 244,234); Neurospora crassa; Schwanniomyces such as Schwanniomyces occidentalis; and filamentous fungi such as, e.g., Neurospora, Penicillium, Tolypocladium, and Aspergillus hosts such as A. nidulans and A. niger.

Suitable host cells for the expression of glycosylated anti-IL-23A antibody are derived from multicellular organisms. Examples of invertebrate cells include plant and insect cells, including, e.g., numerous baculoviral strains and variants and corresponding permissive insect host cells from hosts such as Spodoptera frugiperda (caterpillar), Aedes aegypti (mosquito), Aedes albopictus (mosquito), Drosophila melanogaster (fruitfly), and Bombyx mori (silk worm). A variety of viral strains for transfection are publicly available, e.g., the L-1 variant of Autographa californica NPV and the Bm-5 strain of Bombyx mori NPV, and such viruses may be used, particularly for transfection of Spodoptera frugiperda cells.

Plant cell cultures of cotton, corn, potato, soybean, petunia, tomato, and tobacco can also be utilized as hosts.

In another aspect, expression of anti-IL-23A antibodies is carried out in vertebrate cells. The propagation of vertebrate cells in culture (tissue culture) has become routine procedure and techniques are widely available. Examples of useful mammalian host cell lines are monkey kidney CV1 line transformed by SV40 (COS-7, ATCC CRL 1651), human embryonic kidney line (293 or 293 cells subcloned for growth in suspension culture, (Graham et al., 1977, J. Gen Virol. 36: 59), baby hamster kidney cells (BHK, ATCC CCL 10), Chinese hamster ovary cells/-DHFR1 (CHO, Urlaub et al., 1980, Proc. Natl. Acad. Sci. USA 77: 4216; e.g., DG44), mouse sertoli cells (TM4, Mather, 1980, Biol. Reprod. 23:243-251), monkey kidney cells (CV1 ATCC CCL 70), African green monkey kidney cells (VERO-76, ATCC CRL-1587), human cervical carcinoma cells (HELA, ATCC CCL 2), canine kidney cells (MDCK, ATCC CCL 34), buffalo rat liver cells (BRL 3A, ATCC CRL 1442), human lung cells (W138, ATCC CCL 75), human liver cells (Hep G2, HB 8065), mouse mammary tumor (MMT 060562, ATCC CCL51), TR1 cells (Mather et al., 1982, Annals N.Y. Acad. Sci. 383: 44-68), MRC 5 cells, FS4 cells, and human hepatoma line (Hep G2).

Host cells are transformed with the above-described expression or cloning vectors for anti-IL-23A antibody production and cultured in conventional nutrient media modified as appropriate for inducing promoters, selecting transformants, or amplifying the genes encoding the desired sequences.

The host cells used to produce an anti-IL-23A antibody described herein may be cultured in a variety of media. Commercially available media such as Ham's F10 (Sigma-Aldrich Co., St. Louis, Mo.), Minimal Essential Medium ((MEM), (Sigma-Aldrich Co.), RPMI-1640 (Sigma-Aldrich Co.), and Dulbecco's Modified Eagle's Medium ((DMEM), Sigma-Aldrich Co.) are suitable for culturing the host cells. In addition, any of the media described in one or more of Ham et al., 1979, Meth. Enz. 58: 44, Barnes et al., 1980, Anal. Biochem. 102: 255, U.S. Pat. No. 4,767,704, U.S. Pat. No. 4,657,866, U.S. Pat. No. 4,927,762, U.S. Pat. No. 4,560,655, U.S. Pat. No. 5,122,469, WO 90/103430, and WO 87/00195 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as gentamicin), trace elements (defined as inorganic compounds usually present at final concentrations in the micromolar range), and glucose or an equivalent energy source. Other supplements may also be included at appropriate concentrations that would be known to those skilled in the art. The culture conditions, such as temperature, pH, and the like, are those previously used with the host cell selected for expression, and will be apparent to the ordinarily skilled artisan.

When using recombinant techniques, the antibody can be produced intracellularly, in the periplasmic space, or directly secreted into the medium. If the antibody is produced intracellularly, the cells may be disrupted to release protein as a first step. Particulate debris, either host cells or lysed fragments, can be removed, for example, by centrifugation or ultrafiltration. Carter et al., 1992, Bio/Technology 10:163-167 describes a procedure for isolating antibodies that are secreted to the periplasmic space of E. coli. Briefly, cell paste is thawed in the presence of sodium acetate (pH 3.5), EDTA, and phenylmethylsulfonylfluoride (PMSF) over about 30 minutes. Cell debris can be removed by centrifugation. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants. A variety of methods can be used to isolate the antibody from the host cell.

The antibody composition prepared from the cells can be purified using, for example, hydroxylapatite chromatography, gel electrophoresis, dialysis, and affinity chromatography, with affinity chromatography being a typical purification technique. The suitability of protein A as an affinity ligand depends on the species and isotype of any immunoglobulin Fc domain that is present in the antibody. Protein A can be used to purify antibodies that are based on human gamma1, gamma2, or gamma4 heavy chains (see, e.g., Lindmark et al., 1983 J. Immunol. Meth. 62:1-13). Protein G is recommended for all mouse isotypes and for human gamma3 (see, e.g., Guss et al., 1986 EMBO J. 5:1567-1575). A matrix to which an affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a C_{H3} domain, the Bakerbond ABX™ resin (J. T. Baker, Phillipsburg, N.J.) is useful for purification. Other techniques for protein purification such as fractionation on an ion-exchange column, ethanol precipitation, reverse phase HPLC, chromatography on silica, chromatography on heparin SEPHAROSE™ chromatography on an anion or cation exchange resin (such as a polyaspartic acid column), chromatofocusing, SDS-PAGE, and ammonium sulfate precipitation are also available depending on the antibody to be recovered.

Following any preliminary purification step(s), the mixture comprising the antibody of interest and contaminants may be subjected to low pH hydrophobic interaction chromatography using an elution buffer at a pH between about 2.5-4.5, typically performed at low salt concentrations (e.g., from about 0-0.25M salt).

### Therapeutic Uses

In another embodiment, an anti-IL-23A antibody disclosed herein is useful in the treatment of various disorders associated with the expression of IL-23p19 as described herein. In one aspect, a method for treating an IL-23 associated disorder comprises administering a therapeutically effective amount of an anti-IL-23A antibody to a subject in need thereof.

The anti-IL-23A antibody is administered by any suitable means, including parenteral, subcutaneous, intraperitoneal, intrapulmonary, and intranasal, and, if desired for local immunosuppressive treatment, intralesional administration (including perfusing or otherwise contacting the graft with the antibody before transplantation). The anti-IL-23A antibody or agent can be administered, for example, as an infusion or as a bolus. Parenteral infusions include intramuscular, intravenous, intraarterial, intraperitoneal, or subcutaneous administration. In addition, the anti-IL-23A antibody is suitably administered by pulse infusion, particularly with declining doses of the antibody. In one aspect, the dosing is given by injections, most preferably intravenous or subcutaneous injections, depending in part on whether the administration is brief or chronic. In one aspect, the dosing of the anti-IL-23 antibody is given by subcutaneous injections.

For the prevention or treatment of disease, the appropriate dosage of antibody will depend on a variety of factors such as the type of disease to be treated, as defined above, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments.

The term "suppression" is used herein in the same context as "amelioration" and "alleviation" to mean a lessening of one or more characteristics of the disease.

The antibody is formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "therapeutically effective amount" of the antibody to be administered will be governed by such considerations.

The antibody may optionally be formulated with one or more agents currently used to prevent or treat the disorder in question. The effective amount of such other agents depends on the amount of anti-IL-23A antibody present in the formulation, the type of disorder or treatment, and other factors discussed above.

In one aspect, anti-IL-23A antibodies are useful for treating or preventing a disorder characterized by abnormal expression of IL-23, e.g., by inappropriate activation of immune cells (e.g., lymphocytes or dendritic cells). Such abnormal expression of IL-23 can be due to, for example, increased IL-23 protein levels.

In one aspect, in the context of the present invention, the disease is asthma, in particular severe persistent asthma. Efficacy of treatment of severe persistent asthma in clinical trials is frequently gauged by the time to first asthma worsening (e.g. in days) during the treatment period. Other assessments of efficacy are for example annualized rate of asthma worsening during the treatment period, annualized rate of severe asthma exacerbation during the treatment period, weekly ACQ₅ (Asthma Control Questionnaire) score at a certain time point, e.g. week 24, trough FEV₁ in-clinic change from baseline at a certain time point, e.g. week 24, post-bronchodilator FEV₁ in-clinic change from baseline at a certain time point, e.g. week 24, or time to first severe asthma exacerbation during the treatment period. In one aspect, any one of the above is used to assess the efficacy of an anti-IL23A antibody, for example Antibody A, Antibody B, Antibody C or Antibody D, in the treatment of severe persistent asthma.

### Pharmaceutical Compositions and Administration Thereof

A composition comprising an anti-IL-23A antibody can be administered to a subject having or at risk of having an immunological disorder. The term "subject" as used herein means any mammalian patient to which an anti-IL-23A antibody can be administered, including, e.g., humans and non-human mammals, such as primates, rodents, and dogs. Subjects specifically intended for treatment using the methods described herein include humans. The antibodies can be administered either alone or in combination with other compositions in the prevention or treatment of the respiratory disorder.

Examples of anti-IL-23A antibodies for use in such pharmaceutical compositions are described herein, for example Antibody A, Antibody B, Antibody C or Antibody D.

Various delivery systems are known and can be used to administer the anti-IL-23A antibody. Methods of introduction include but are not limited to intradermal, intramuscular, intraperitoneal, intravenous, subcutaneous, intranasal, epidural, and oral routes. The anti-IL-23A antibody can be administered, for example by infusion, bolus or injection, and can be administered together with other biologically active agents such as chemotherapeutic agents. Administration can be systemic or local. In one embodiment, the administration is by subcutaneous injection. Formulations for such injections may be prepared in for example prefilled syringes that may be administered once every other week.

In specific embodiments, the anti-IL-23A antibody is administered by injection, by means of a catheter, by means of a suppository, or by means of an implant, the implant being of a porous, non-porous, or gelatinous material, including a membrane, such as a sialastic membrane, or a fiber. Typically, when administering the composition, materials to which the anti-IL-23A antibody or agent does not absorb are used.

In other embodiments, the anti-IL-23A antibody is delivered in a controlled release system. In one embodiment, a pump may be used (see, e.g., Langer, 1990, Science 249:1527-1533; Sefton, 1989, CRC Crit. Ref. Biomed. Eng. 14:201; Buchwald et al., 1980, Surgery 88:507; Saudek et al., 1989, N. Engl. J. Med. 321:574). In another embodiment, polymeric materials can be used. (See, e.g., Medical Applications of Controlled Release (Langer and Wise eds., CRC Press, Boca Raton, Fla., 1974); Controlled Drug Bioavailability, Drug Product Design and Performance (Smolen and Ball eds., Wiley, New York, 1984); Ranger and Peppas, 1983, Macromol. Sci. Rev. Macromol. Chem. 23:61. See also Levy et al., 1985, Science 228:190; During et al., 1989, Ann. Neurol. 25:351; Howard et al., 1989, J. Neurosurg. 71:105.) Other controlled release systems are discussed, for example, in Langer, supra.

An anti-IL-23p19 antibody is typically administered as pharmaceutical compositions comprising a therapeutically effective amount of the antibody and one or more pharmaceutically compatible ingredients.

In typical embodiments, the pharmaceutical composition is formulated in accordance with routine procedures as a pharmaceutical composition adapted for intravenous or subcutaneous administration to human beings. Typically, compositions for administration by injection are solutions in sterile isotonic aqueous buffer. Where necessary, the pharmaceutical can also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the pharmaceutical is to be administered by infusion, it can be dispensed with an infusion bottle containing sterile pharmaceutical grade water or saline. Where the pharmaceutical is administered by injection, an ampoule of sterile water for injection or saline can be provided so that the ingredients can be mixed prior to administration.

Further, the pharmaceutical composition can be provided as a pharmaceutical kit comprising (a) a container containing an anti-IL-23A antibody in lyophilized form and (b) a second container containing a pharmaceutically acceptable diluent (e.g., sterile water) for injection. The pharmaceutically acceptable diluent can be used for reconstitution or dilution of the lyophilized anti-IL-23A antibody. Optionally associated with such container(s) can be a notice in the form prescribed by a governmental agency regulating the manufacture, use or sale of pharmaceuticals or biological products, which notice reflects approval by the agency of manufacture, use or sale for human administration.

In one aspect, 90 mg of the anti-IL23A antibody, for example Antibody A, Antibody B, Antibody C or Antibody D, is administered to a patient, for example once every 4 weeks. The anti-IL-23A antibody is for example administered to the patient at week 0, at week 4, at week 8, at week 12, at week 16, at week 20 and thereafter at 4 weeks intervals. In one aspect, the administration is by subcutaneous injection.

Examples of pharmaceutical compositions used in the context of the present invention are disclosed in Example 3 herein below.

The invention is further described in the following examples, which are not intended to limit the scope of the invention.

### Examples

### Example 1a: Pharmacological studies

To study downstream signals of IL-23 receptor activation rat precision cut lung slices were used.

Lungs were taken from male Wistar rats (250 - 400 g) and kept under controlled conditions (22° C, 55% humidity, 12-h day/night rhythm). To obtain the lung slices rats were anesthetized by intraperitoneal injection of 60 mg/kg pentobarbital sodium (Narcoren, Merial GmbH, Germany). After exsanguination and death of the animal, the lung was filled with 1.5% low-melting agarose (Sigma Aldrich) in situ in medium and covered with ice for 10 minutes to allow the agarose to cool and solidify. The lung was removed from the thoracic cavity and the lobes were separated. Cylinders (diameter: 8mm) were punched out with a coring tool and cut with a Krumdieck tissue slicer (Alabama Research and Development, Munford, US) into 250 µm thin horizontal slices. Lung slices were incubated in medium at 37°C, 5% CO2, and 95% air humidity under cell culture conditions. The medium used for incubation contained MEM (Minimum Essential Media, Life Technologies) was supplemented with following components (supplier, final concentration). Glucose (B.Braun, 17mM), NaHCO3 (Sigma, 26mM), Hepes (Gibco, 25mM), Sodium pyruvate (Gibco, 1mM), GlutaMax (Gibco, 2mM), Penicillin-Streptomycin (Sigma, 1%v/v).

Stimulation was performed with IL-23. Cytokine release was measured with ELISAs from R&D Systems.

IL-23 (10 or 100 nM) was added to the slices. The slices were incubated at 37°C, 5% CO₂, and 95% air humidity and rotated at 1 rpm for the indicated time points. After the incubation (time points given below) culture supernatants were collected and stored at -80 °C until the ELISA was performed. 6 slices were used per data point. At baseline these tissue slices do not express neutrophil markers on the mRNA levels or do not express typical markers of Th17 cells.

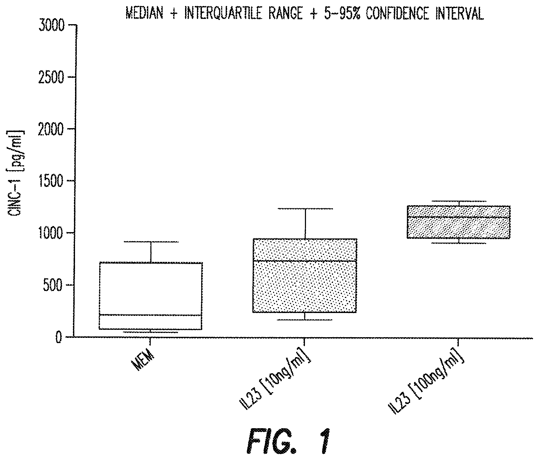

Figure 1 shows that the treatment of rat precision-cut lung slices with IL-23 drives a fast release of the neutrophil attractant Cxcl1 (CINC1) after 2hrs after start of the stimulation.

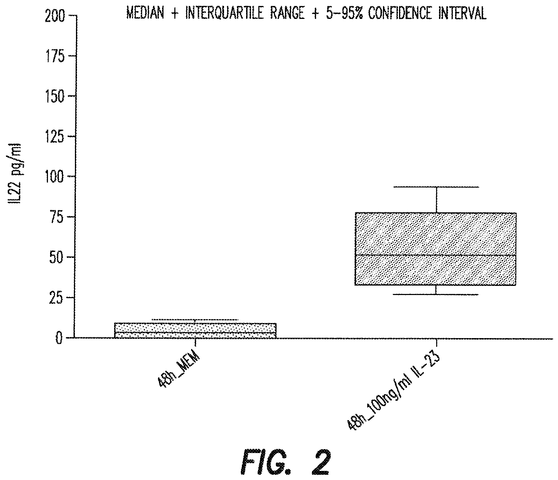

Figure 2 shows that the treatment of rat precision-cut lung slices with IL-23 drives the release of IL22 48 hrs after start of the stimulation.

### Example 1b: Pharmacological studies

Precision-cut lung slices (PCLS) were prepared from adult male Wistar rats as briefly described below. Rats were housed under conventional conditions in isolated ventilated cages with free access to water and food.

Rat lungs or human lobes were filled via the trachea and lobular bronchus, respectively, with 1.5% low-melting agarose dissolved in Williams minimal essential medium (MEM) and cooled on ice for gelling. Tissue cores (8mm in diameter) were punched out and cut into PCLS by means of a Krumdieck Tissue Slicer. PCLS were harvested and kept under standard cell culture conditions (MEM, 37°C, 5% CO₂). Medium was changed every 30 min during the first 4h after preparation and then the next day. For challenge PCLS were transferred to 48-well plates (1 PCLS/500µL). Challenges comprised IL23 (100 ng/mL or 300 ng/mL) for 24 or 48 h or were left untreated (control). In human PCLS, IL17 (100 ng/mL or 300 ng/mL) was additionally tested.

### Measurement of IL22

The cytokine IL22 was measured in supernatants from PCLS treatments. For rat, ELISA (R&D, M2200) was performed according to the manufacturer's instructions. Human IL22 was determined by high-sensitive Singulex assay.

### Statistics:

Data analysis was performed using GraphPad Prism 6. Equality of variances was checked by the Bartlett's test. If variances were equal, group means were compared by ANOVA followed by Dunnett's multiple comparison test. If variances were unequal, group means were compared by Kruskal-Wallis test followed by Dunn's post-test to correct for multiple comparisons. Groups were considered significantly different, if p-value was smaller than 0.05.

### Results

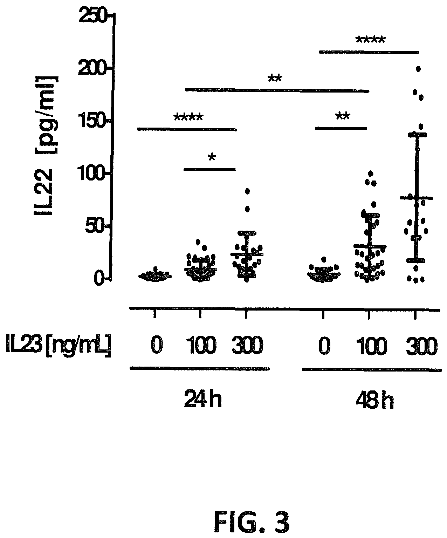

To screen for pulmonary biomarkers involved in IL23 signaling, PCLS were challenged with IL23 and released cytokines (TNFα, IL1β, IL6, IL8, IL13, IL17, IL21 and IL22) were quantified. In rat PCLS, only IL22 was reproducibly induced in a concentration- and time-dependent manner (figure 3).

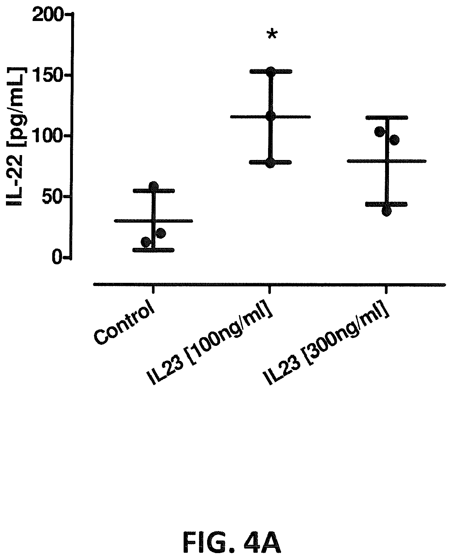

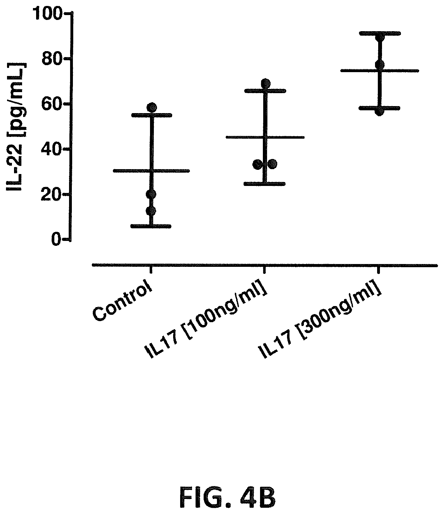

Supernatants from IL23 challenged human PCLS were analyzed for IL17A/F, IL22 and β2-defensin protein release. Again, only IL22 arose significantly after 24h IL23 treatment (figure 4A). In contrast, IL17 did not induce IL22 (figure 4B).

### Example 2: Clinical study

Antibody A is administered subcutaneously to patients with severe persistent asthma in a randomized, double-blind, placebo controlled, parallel group study to assess its safety and efficacy as add-on therapy over 24 weeks. 90 mg of Antibody A is administered once every 4 weeks at weeks 0, 4, 8, 12, 16 and 20.

The inclusion criteria in the study are the following:
1. Pre-bronchodilator clinic measured FEV1 of =40% and =85% of predicted normal (FEV1 = Forced Expiratory Volume in 1 second).
2. One year history of asthma diagnosed by a physician, and have FEV1 reversibility of = 12% and an absolute change of at least 200 mL after administration of 400 µg salbutamol.
3. Must be on at least medium dose inhaled corticosteroids and at least one other asthma controller medication for at least one year.
4. Must have documented history of two or more severe asthma exacerbations in the last 12 months.

The efficacy of Antibody A is assessed by one or more of the following endpoints, which are determined using known methods.

The primary endpoint is the time to first asthma worsening during the planned 24 week treatment period for active vs. placebo treated patients on top of standard of care therapy.

Additional endpoints are:
- Annualized rate of asthma worsening during the planned 24 week treatment period.
- Trough FEV1 in-clinic change from baseline at week 24.
- Annualized rate of severe asthma exacerbation during the planned 24-week treatment period.
- Post-bronchodilator FEV1 in-clinic change from baseline at week 24.
- Time to first severe asthma exacerbation during the planned 24 week treatment period.
- Weekly Asthma Control Questionaire score at week 24.

### Example 3: Pharmaceutical compositions

Examples of formulations suitable for an antibody of the present invention are shown below. Antibodies used in the formulations below are for example Antibody A, Antibody B, Antibody C or Antibody D.

### Formulation 1:

| **Components** | **Concentration [mmol/L]** | **Concentration [g/l]** | **Nominal Amount [mg/vial] V = 10.0 ml** |
|---|---|---|---|
| Antibody | | 10.0 | 100.0 |
| Succinic acid | 0.7 | 0.083 | 0.8 |
| Disodium succinate hexahydrate | 24.3 | 6.564 | 65.6 |
| Sodium chloride | 125 | 7.305 | 73.1 |
| Polysorbat 20 | 0.16 | 0.20 | 0.20 |
| Water for Injection | - | Ad 1L | Ad 1mL |

The pH of formulation 1 is typically in the range of pH 6.0 to 7.0, for example pH 6.5. This formulation is particularly suitable for intravenous administration.

Molecular weight (MW in g/mol) of used excipients: Disodium succinate hexahydrate = 270.14 g/mol; Succinic acid = 118.09 g/mol; Sodium chloride = 58.44 g/mol.

The osmolarity of the formulation is 300 +/- 30 mOsmol/kg, as determined using an Osmomat 030 (Gonotec GmbH, Berlin, Germany). The density at 20°C of the formulation is approximately 1.0089 g/cm³, as determined using a measuring unit DMA 4500 (Anton Paar GmbH, Ostfildern-Scharnhausen, Germany).

### Formulation 2:

| **Components** | **Concentration [mmol/L]** | **Concentration [g/l]** | **Nominal Amount [mg/syringe] V = 1.0 ml** |
|---|---|---|---|
| Antibody | 0.6 | 90.0 | 90.0 |
| Succinic acid | 0.5 | 0.059 | 0.059 |
| Disodium succinate hexahydrate | 3.9 | 1.054 | 1.054 |
| Sorbitol | 225 | 41.00 | 41.00 |
| Polysorbat 20 | 0.16 | 0.20 | 0.20 |
| Water for Injection | - | Ad 1L | Ad 1mL |

The pH of formulation 2 is typically in the range of pH 5.5 to 6.5, for example 5.5 to 6.1, for example the pH is 5.8. This formulation is particularly suitable for subcutaneous administration.

Molecular weight (MW in g/mol) of used excipients:
MW: Succinic acid (C₄H₆O₄)= 118.09 g/mol
MW: Disodium succinate hexahydrate (C₄O₄Na₂H4 x 6H₂O) = 270.14 g/mol
MW: Sorbitol = 182.17 g/mol
MW: Polysorbate 20 = 1227.72 g/mol

The osmolarity of the formulation is 300 +/- 30 mOsmol/kg, as determined using an Osmomat 030 (Gonotec GmbH, Berlin, Germany). The density at 20°C of the formulation is approximately 1.040 g/cm³, as determined using a measuring unit DMA 4500 (Anton Paar GmbH, Ostfildern-Scharnhausen, Germany).

### Formulation 3:

| **Components** | **Concentration [mmol/L]** | **Concentration [g/l]** | **Nominal Amount [mg/syringe] V = 1.0 ml** |
|---|---|---|---|
| Antibody | 0.6 | 90.0 | 90.0 |
| Sorbitol | 240 | 43.733 | 43.733 |
| Polysorbat 20 | 0.16 | 0.20 | 0.20 |
| Water for Injection | - | Ad 1L | Ad 1mL |

The pH of formulation 3 is typically in the range of pH 5.5 to 6.5, for example 5.5 to 6.1, for example the pH is 5.8. This formulation is particularly suitable for subcutaneous administration.

Molecular weight (MW in g/mol) of used excipients:
MW: Sorbitol = 182.17 g/mol
MW: Polysorbate 20 = 1227.72 g/mol.

The osmolarity of the formulation is 300 +/- 30 mOsmol/kg, as determined using an Osmomat 030 (Gonotec GmbH, Berlin, Germany).

### Preferred Embodiments

1. A method for treating severe persistent asthma comprising administering to a patient an anti-IL-23A antibody.
2. The method according to item 1, wherein said patient is not responsive to corticosteroids.
3. The method according to any one of items 1 or 2, wherein said anti-IL-23A antibody is Antibody A, Antibody B, Antibody C or Antibody D.
4. The method according to any one of items 1 to 3, wherein said anti-IL-23A antibody is administered to said patient by subcutaneous administration.
5. The method according to any one of items 1 to 4, wherein said asthma is neutrophilic asthma, eosinophilic asthma, mixed neutrophilic and eosinophilic asthma or paucigranulocytic asthma.
6. The method according to any one of items 1 to 5, wherein the anti-IL-23A antibody comprises a light chain comprising the amino acid sequence of SEQ ID NO:18 and a heavy chain comprising the amino acid sequence of SEQ ID NO:19.
7. The method according to any one of items 1 to 5, wherein the anti-IL-23A antibody comprises a light chain comprising the amino acid sequence of SEQ ID NO:18 and a heavy chain comprising the amino acid sequence of SEQ ID NO:20.
8. The method according to any one of items 1 to 5, wherein the anti-IL-23A antibody comprises a light chain comprising the amino acid sequence of SEQ ID NO:21 and a heavy chain comprising the amino acid sequence of SEQ ID NO:19.
9. The method according to any one of items 1 to 5, wherein the anti-IL-23A antibody comprises a light chain comprising the amino acid sequence of SEQ ID NO: 21 and a heavy chain comprising the amino acid sequence of SEQ ID NO: 20.

## Claims

1. An anti-IL-23A antibody for use in a method for treating severe persistent asthma.

2. The antibody for the use according to claim 1, wherein said patient is not responsive to corticosteroids.

3. The antibody for the use according to any one of claims 1 or 2, wherein said anti-IL-23A antibody is Antibody A, Antibody B, Antibody C or Antibody D.

4. The antibody for the use according to any one of claims 1 to 3, wherein said anti-IL-23A antibody is to be administered to said patient by subcutaneous administration.

5. The antibody for the use according to any one of claims 1 to 4, wherein said asthma is neutrophilic asthma, eosinophilic asthma, mixed neutrophilic and eosinophilic asthma or paucigranulocytic asthma.

6. The antibody for the use according to any one of claims 1 to 5, wherein the anti-IL-23A antibody comprises a light chain comprising the amino acid sequence of SEQ ID NO:18 and a heavy chain comprising the amino acid sequence of SEQ ID NO:19.

7. The antibody for the use according to any one of claims 1 to 5, wherein the anti-IL-23A antibody comprises a light chain comprising the amino acid sequence of SEQ ID NO:18 and a heavy chain comprising the amino acid sequence of SEQ ID NO:20.

8. The antibody for the use according to any one of claims 1 to 5, wherein the anti-IL-23A antibody comprises a light chain comprising the amino acid sequence of SEQ ID NO:21 and a heavy chain comprising the amino acid sequence of SEQ ID NO:19.

9. The antibody for the use according to any one of claims 1 to 5, wherein the anti-IL-23A antibody comprises a light chain comprising the amino acid sequence of SEQ ID NO: 21 and a heavy chain comprising the amino acid sequence of SEQ ID NO: 20.
